(19) Europäisches Patentamt
European Patent Office
Office européen des brevets

(11) Veröffentlichungsnummer: **0 173 033**
**B1**

# (12) EUROPÄISCHE PATENTSCHRIFT

(45) Veröffentlichungstag der Patentschrift: **31.01.90**

(21) Anmeldenummer: **85108345.1**

(22) Anmeldetag: **05.07.85**

(51) Int. Cl.[4]: **C 08 F 220/56,** C 08 F 220/06 // (C08F220/56, 220:06),(C08F230/06, 220:56)

## (54) Verfahren zur Herstellung von Copolymeren.

(30) Priorität: **24.07.84 DE 3427220**

(43) Veröffentlichungstag der Anmeldung: **05.03.86 Patentblatt 86/10**

(45) Bekanntmachung des Hinweises auf die Patenterteilung: **31.01.90 Patentblatt 90/5**

(84) Benannte Vertragsstaaten: **AT BE CH DE FR GB LI**

(56) Entgegenhaltungen: **EP-A- 0 037 378**
**DE-A- 3 221 284**

**Die Akte enthält technische Angaben, die nach dem Eingang der Anmeldung eingereicht wurden und die nicht in dieser Patentschrift enthalten sind.**

(73) Patentinhaber: **CASSELLA Aktiengesellschaft, Hanauer Landstrasse 526, D-6000 Frankfurt am Main 61 (DE)**

(72) Erfinder: **Sauer, Josef, Dr., Rannenbergring 9, D-8755 Alzenau (DE)**
Erfinder: **Engelhardt, Friedrich, Dr., Hünfelder Strasse, D-6000 Frankfurt/Main 61 (DE)**
Erfinder: **Rabas, Kerstine, Hanauer Landstrasse 513a, D-6000 Frankfurt/Main 61 (DE)**
Erfinder: **Quack, Jochen M. Dr., Wilhelm-Reuter-Strasse 16, D-6239 Eppstein 3 (DE)**
Erfinder: **Reng, Alwin K. DI, Im Schulzehnten 22, D-6233 Kelkheim 2 (DE)**
Erfinder: **Skzypzak, Werner, DI, Eichkopfallee 33, D-6237 Liederbach (DE)**

(74) Vertreter: **Urbach, Hans-Georg, Dr. et al, Hanauer Landstrasse 526, D-6000 Frankfurt am Main 61 (DE)**

EP 0 173 033 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von Copolymeren aus Acrylamid, Acrylsäure und/oder Methacrylsäure und ihren Salzen sowie gegebenenfalls einem Vernetzer und weiteren copolymerisierbaren Monomeren, bei denen ein Teil des Monomerengemisches im Reaktionsraum vorgelegt und, nach Anspringen der Polymerisation, der Rest der Monomeren in den Reaktionsraum eindosiert wird.

Das Verfahren gestattet aufgrund seiner Variabilität die gezielte Herstellung einer breiten Palette von Copolymerisaten mit spezifischen rheologischen Eigenschaften sowie insbesondere verbesserter Elektrolytstabilität. Die so erhaltenen Copolymere eignen sich hervorragend als Verdickungsmittel für wäßrige Systeme auch in Gegenwart von Elektrolyten.

Copolymere aus Acrylsäure und einem Polyallylether eines Polyols mit wenigstens vier C-Atomen und drei OH-Gruppen im Molekül als Vernetzungskomponente sind als Verdickungsmittel bekannt. Sie werden nach DE-C-1 042 233 durch Fällungspolymerisation in Benzol, Xylol, Tetralin, Hexan, Heptan, Tetrachlorkohlenstoff, Methylchlorid, Ethylchlorid und Bromtrichlormethan, sowie Mischungen dieser und anderer nicht namentlich genannter Lösungsmittel hergestellt. In DP-1 042 233 werden auch Copolymerisate der Acrylsäure mit weiteren mischpolymerisierbaren Monomeren beschrieben, wobei N-Methylacrylamid und Styrol als Comonomere namentlich erwähnt werden. Das Verfahren hat jedoch den Nachteil, daß bei Verwendung der oben angegebenen unpolaren Lösungsmittel über 8–10%ige Polyacrylsäuresuspensionen nicht mehr gerührt werden können.

In US-PS-2 980 655 wird eine Verbesserung des oben angegebenen Polymerisationsverfahrens vorgeschlagen in der Hinsicht, daß durch Zusatz «polarer» Lösungsmittel zum bevorzugt eingesetzten Lösungsmittel Benzol (bevorzugte polare Lösungsmittel sind Alkohole mit nicht mehr als 4 C-Atomen) ein geringeres Anschwellen des Polymers beobachtet wird, so daß auch über 18–20%ige Polyacrylsäuresuspensionen noch gerührt werden können. Die Polymerisationsreaktion wird im geschlossenen Reaktionsgefäß im Batchbetrieb durchgeführt.

Aus der DE-OS-2 927 132 ist ein gegenüber der US-PS-2 980 655 verbessertes Verfahren bekannt zur Herstellung von vernetzter Polyacrylsäure bzw. deren Salzen, bei dem die Polymerisation in einem von aromatischen Kohlenwasserstoffen freien Gemisch bestimmter organischer Lösungsmittel, das einen gewissen Anteil bestimmter sauerstoffhaltiger Lösungsmittel aufweist, nach dem Prinzip der Fällungspolymerisation ausgeführt wird.

Insbesondere wird wegen der Toxizität des Benzols Cyclohexan oder ein aliphatischer gesättigter Kohlenwasserstoff mit 1–5 C-Atomen und 1–4 Chloratomen als unpolare Komponente sowie n-Propanol oder ein gesättigter aliphatischer Monoalkohol mit 4–6 C-Atomen oder ein aliphatischer gesättigter Carbonsäureester oder Methylethylketon als sauerstoffhaltige Komponente empfohlen. Die nach diesem Verfahren erhaltenen vernetzten Polyacrylsäuren bzw. deren Salze bilden in Wasser hochviskose Lösungen und Gele, die jedoch noch eine gewisse störende Körnigkeit und Trübung aufweisen. Um diese Nachteile zu vermeiden, kann dies bekannte Polymerisationsverfahren auch in der Weise ausgeführt werden, daß 50–100 Vol% des Lösungsmittelgemisches zusammen mit 0–100 Gew.% des vernetzenden Monomeren vorgelegt und die Gesamtmenge der Acrylsäure sowie die sich auf 100 Gew.% ergänzende Menge bzw. die Gesamtmenge (100 Gew.%) an vernetzendem Monomeren und die sich auf 100 Vol.% ergänzende Menge des organischen Lösungsmittelgemisches innerhalb von 1 bis 8 Stunden, vorzugsweise 2 bis 6 Stunden, so zu der auf die Polymerisationstemperatur erhitzten Vorlage dosiert werden, daß sich nach Zudosierung von 50 Gew.% der Acrylsäure 60–100 Gew.% des vernetzenden Monomeren im Reaktionsgemisch befinden.

Die EP-Patentanmeldung 69 371 beschreibt ein Verfahren zur Herstellung vernetzter Polyacrylsäuren mit verbessertem – Polymerisationsverhalten. Dem Lösungsmittel für die Fällungspolymerisation (vorzugsweise wird Methylenchlorid eingesetzt) wird ein Tensid mit einem HLB-Wert unter 10 zugesetzt. Dies führt zu einem einheitlicheren Kornspektrum mit verbesserter Rührmöglichkeit, Wärmeabfuhr und geringerem Polymerbelagsaufbau. In US-PS-4 419 502 wird ein Tensid mit einem HLB-Wert über 12 zur Verringerung oder Vermeidung von Polymerwandbelag vorgeschlagen. Auch in dieser Anmeldung wird Methylenchlorid als Lösungsmittel beansprucht.

In DE-OS-3 221 284 wird ein Verfahren zur Herstellung eines pulverförmigen Salzes eines Methacrylsäurepolymerisats durch Fällungspolymerisation in Alkohol beschrieben. Bevorzugt wird Ethylalkohol eingesetzt, als Comonomer auch Acrylamid.

Zur Erzielung bestimmter anwendungstechnischer Eigenschaften ist es häufig erforderlich, verschiedene nach den oben angegebenen bekannten Verfahren hergestellte Copolymerisate, die sich im Molekulargewicht unterscheiden, abzumischen. So empfiehlt beispielsweise eine Firmenschrift der B. F. Goodrich, hochmolekulare Polyacrylsäuren mit niedermolekularen Polyacrylsäuren zu mischen, um bei Verwendung der Polyacrylsäuren als Verdickungsmittel für den Textildruck eine höhere Elektrolytstabilität zu erzielen. Ein ähnliches Verfahren wird in der EP-Anmeldung 77 297 beschrieben. In dieser Druckschrift wird empfohlen ein Copolymerisat aus Acrylamid und Acrylsäure, das mit einer Homo-Polyacrylsäure versetzt ist, als Verdickungsmittel in Druckpasten zum Färben und Bedrucken von Fasermaterialien einzusetzen.

Eine ähnliche Überlegung liegt der EP-Anmeldung 48 612 zugrunde, in der die Abmischung eines elektrolytverträglicheren Gums mit einer Polyacrylsäure als Verdickungsmittel beschrieben wird.

Die bekannten Verfahren zur Herstellung von vernetzten Polyacrylsäuren weisen erhebliche oben geschilderte Nachteile und Schwierigkeiten auf. Produkte mit hoher Viskositätsergiebigkeit und ausreichender Elektrolytstabilität sind erst durch Abmischung von vernetzten Polyacrylsäuren unterschiedlichen Molekulargewichts sowie z. Teil mit anderen Polymeren und Copolymeren zu erhalten. Ferner ist je nach anwendungstechnischem Einsatz eine möglichst gute Anpassung der Polymer-Eigenschaften an den Anwendungszweck erwünscht. So kann für bestimmte Zwecke eine besonders hohe Viskositätsergiebigkeit, für andere Zwecke eine besonders hohe Elektrolytverträglichkeit erforderlich sein, es ist aber auch möglich, wie z. B. beim Einsatz als Verdickungsmittel im Pigmentdruck, daß Viskositätsergiebigkeit und Elektrolytbeständigkeit möglichst optimal aufeinander abgestimmt sind.

Für diesen Einsatzzweck ist es nämlich erforderlich, daß während der Herstellung der Farbstoffdispersion die durch das Polymerisat verursachte Verdickung nicht zu einer zu hohen Viskosität führt, weil dies die Handhabbarkeit der Paste sehr erschwert, die Homogenisierung einen unvertretbar hohen Energieaufwand erfordert und womöglich auch die erzielbare Feinverteilung des Farbstoffs leidet. Andererseits darf nach dem Zusatz des in der Regel elektrolythaltigen Bindemittels (das nicht mit dem Verdicker selbst zu verwechseln ist) die Viskosität nicht unter einen für den Druckvorgang erforderlichen Minimalwert absinken.

Die bisher bekannten Polymerisationsverfahren ermöglichen nicht die Herstellung von Copolymerisaten, insbesondere solcher, die ganz oder überwiegend aus den Monomeren Acrylamid und Acrylsäure bzw. Methacrylsäure aufgebaut sind, die solche speziellen rheologischen Eigenschaften aufweisen.

Es bestand daher ein dringendes Bedürfnis nach einem Polymerisationsverfahren, das es gestattet, bei Einsatz technisch gut zugänglicher Ausgangsmaterialien die Eigenschaften der erhaltenen Polymerisate in einem relativ weiten Bereich zu variieren und dem geplanten Einsatzzweck optimal anzupassen.

Es wurde nun gefunden, daß man die Schwierigkeiten der bekannten Polymerisationsverfahren vermeiden kann und Copolymerisate erhält, deren Eigenschaften durch das Herstellungsverfahren in weiten Grenzen variiert und den Erfordernissen angepaßt werden können, wenn man zur Herstellung von 100 Gew.-Teilen des Copolymerisats

10–90 Gew.-Teile Acrylamid (Monomer I)
90–10 Gew.-Teile Acrylsäure und/oder Methacrylsäure und/oder deren Salze mit dem Kation $M^+$ (Monomer II)
0–40 Gew.-Teile eines copolymerisierbaren Monomeren der Formel III

$$R^3_a\text{–}CH_{2-a}=\underset{\displaystyle X}{\underset{|}{CH_{1-b}}}\text{–}R^3_b \qquad (III)$$

und
0–5 Gew.-Teile eines copolymerisierbaren, zwei oder mehr olefinische Doppelbindungen aufweisenden, bekannten Vernetzers

in tert.-Butanol bei Temperaturen zwischen 20 und 120 °C nach Art einer Fällungspolymerisation so polymerisiert, daß man
10–90 Gew.% des Alkohols
10–90 Gew.% der Gesamtmenge der Monomeren I bis III und
10–100 Gew.% der Vernetzersubstanz
vorlegt, die Polymerisation mit
10–100 Gew.% des Initiators startet und den sich auf 100% ergänzenden Rest von Lösungsmittel, Monomeren, Vernetzer und Initiator nach Anspringen der Polymerisation einzeln oder im Gemisch miteinander im Verlauf von 1–10 Stunden zudosiert.

In der oben angegebenen Formel III der fakultativ einzusetzenden Comonomeren bedeuten
$R^3$ Wasserstoff oder Methyl,
a und b jeweils die Werte 0 oder 1, wobei die Summe a + b ebenfalls 0 oder 1 ist, und
X eine Gruppe der Formel IV

$$\begin{array}{l} | \\ N\text{–}R^4 \\ | \\ COR^5 \end{array} \qquad (IV)$$

worin $R^4$ und $R^5$ unabhängig voneinander für Wasserstoff, Alkyl mit 1 bis 4 Kohlenstoffatomen oder gemeinsam für Trimethylen oder Pentamethylen stehen und wobei für $R^4$ auch Methylol stehen kann, sofern $R^5$ Alkyl ist; Alkoxycarbonyl mit 1 bis 20 Kohlenstoffatomen; Hydroxyalkoxycarbonyl mit 1 bis 3 Kohlenstoffatomen; N-Methylolcarbonamid der Formel
$HOCH_2NH\text{–}CO\text{–}$,
dessen Methylolgruppe gegebenenfalls mit Alkanolen mit 1 bis 4 Kohlenstoffatomen verethert sein kann; Cyan; Phenyl oder Benzyl, mit 1 oder 2 Substituenten substituiertes Phenyl oder Benzyl; Imidazolyl-(1); die Sulfonsäuregruppe; Sulfoalkylamidocarbonyl mit 1 bis 4 Kohlenstoffatomen im Alkylrest; die Phosphonsäuregruppe, wobei die Sulfonsäure- und Phosphonsäuregruppen auch in Form ihrer Salze mit einem Kation $M^+$ vorliegen können; die Phosphonsäureestergruppe der Formel V

$$\begin{array}{l} \;\;O \\ \;\;\| \\ \text{–}P\text{–}OR^6 \\ \;\;| \\ \;\;O^{\ominus}M^{\oplus} \end{array} \qquad (V)$$

worin $R^6$ Alkyl mit 1 bis 4 Kohlenstoffatomen ist; einen Rest der Formel VI

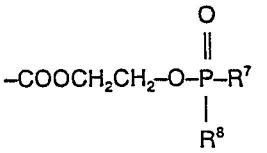 (VI)

worin $R^7$ und $R^8$ gleich oder verschieden sind und für Alkyl mit 1 bis 7 Kohlenstoffatomen stehen; einen Rest der Formel VII

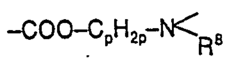 (VII)

worin $R^7$ und $R^8$ die oben angegebenen Bedeutungen haben und p für eine Zahl von 1 bis 4 steht; oder einen Rest der Formel VIII

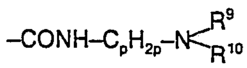 (VIII)

worin $R^9$ oder $R^{10}$ gleich oder verschieden sind und für Alkyl mit 1 bis 4 Kohlenstoffatomen stehen und p für eine Zahl von 1 bis 4 steht; sowie die den Formeln VII und VIII entsprechenden quaternisierten Gruppen, wobei mindestens 50% der Grundkettenbausteine solche Reste X aufweisen, die hydrophilen Charakter besitzen und mindestens etwa 2% der Reste X saure Gruppen aufweisen bzw. deren Salze mit dem Kation $M^+$, wobei sich $M^+$ prinzipiell von jeder wasserlöslichen bekannten Base ableiten kann, deren Stärke ausreicht, die Sulfongruppen bzw. Carboxylgruppen der erfindungsgemäßen vernetzten Copolymerisate zu neutralisieren und die deren Hydrophilie nicht beeinträchtigt.

Für $R^7$, $R^8$, $R^9$ oder $R^{10}$ stehende Alkylreste haben bevorzugt 1 oder 2 Kohlenstoffatome.

Die den Formeln VII und VIII entsprechenden quaternisierten Gruppen sind beispielsweise durch Dimethylsulfat oder Methylchlorid quaternisiert.

Geeignete gängige Substituenten für einen für X stehenden Phenyl- oder Benzylrest sind Halogen und Alkyl mit 1 bis 4 C-Atomen, insbesondere Chlor, Methyl und Ethyl. Einfachsubstitution kann in o-, m- oder p-Stellung zur Vinyl- oder Allylgruppe vorliegen, Zweifachsubstitution, vorzugsweise in 2,4- oder 2,6-Stellung, aber auch in 2,5-, 3,5- oder 3,4-Stellung.

Bevorzugt sind, sofern X Phenyl oder Benzyl bedeutet, die im aromatischen Kern nur einfach substituierten und besonders bevorzugt die unsubstituierten Reste.

Weitere Substituenten, die ein für X stehender Phenyl- oder Benzylrest tragen kann, sind Alkoxy mit 1 oder 2 C-Atomen, Fluor, Trifluormethyl oder Nitro.

Typische Reste X, die hydrophilen Charakter haben, sind der Sulfonsäurerest oder Carboxyl sowie Gruppen, die diese sauren Reste tragen, Carbonamid ($-CO-NH_2$) sowie dessen Methylolderivat, und die Gruppe der Formel IV.

Typische Reste X ohne hydrophilen Charakter sind z. B. Cyan, Phenyl oder Benzyl.

Vorzugsweise weisen mindestens 7% der Reste X saure Gruppen bzw. deren Salze mit dem Kation $M^+$ auf. Die Auswahl des Kations $M^+$ kann in einfacher bekannter Weise erfolgen.

Zweckmäßigerweise bedeutet $M^+$ ein Erdalkali- oder vorzugsweise ein Alkalikation, insbesondere ein Natrium- oder Kaliumkation, Ammonium oder ein von niederen aliphatischen Aminen abgeleitetes Kation. Niedere aliphatische Amine, von denen sich die Kationen $M^+$ ableiten können, sind primär, sekundär oder tertiär und weisen gegebenenfalls durch –OH-Gruppen substituierte Alkylgruppen mit 1 bis 4 C-Atomen auf. Bevorzugt sind solche, die mindestens einen β-Hydroxyethylrest enthalten, wie z. B. β-Aminoethanol, β-Dimethylamino-ethanol, Bis-(β-hydroxyethyl)-methylamin, Tris-(β-hydroxyethyl)-amin, Diethyl-β-hydroxyethylamin, Bis-(β-hydroxyethyl)-ethylamin.

Bevorzugt als fakultative einzusetzende Comonomere sind solche der Formel III, in denen a = 0 und b = 1 ist, die somit der Formel IX

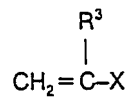 (IX)

entsprechen und in denen $R^3$ Wasserstoff oder Methyl ist und

X eine Gruppe der Formel IV

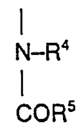 (IV)

worin $R^4$ und $R^5$ unabhängig voneinander für Wasserstoff, Methyl oder Ethyl oder gemeinsam für Trimethylen oder Pentamethylen stehen; Alkoxycarbonyl mit 1 bis 20, vorzugsweise 1 bis 4, C-Atomen, Hydroxyalkoxycarbonyl mit 2 oder 3, vorzugsweise 2, C-Atomen; die Sulfonsäuregruppe; Sulfoalkylamidocarbonyl mit 1 bis 4 C-Atomen im Alkylrest, vorzugsweise eine Gruppe der Formel

$$CO-NH-C(CH_3)_2-CH_2-SO_3^{\ominus}M^{\oplus},$$

die Phosphonsäuregruppe, wobei die Sulfonsäure- und Phosphonsäuregruppen auch in Form ihrer Alkali- oder Ammoniumsalze vorliegen können; und die Phosphonsäureestergruppe der Formel V

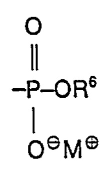 (V)

worin $R_6$ die obengenannten Bedeutungen hat, bedeutet.

Beispiele für copolymerisierbare Monomere der Formeln III bzw. IX sind N-Vinyl-N-methylacetamid, 2-Acrylamido-2-methyl-propansulfonsäure, Vinylphosphonsäure, Vinylphosphonsäureanhydrid, Ester der Acrylsäure oder Methacrylsäure mit Alkanolen mit 1–4 C-Atomen.

Als Vernetzer werden mehrfach olefinisch ungesättigte Verbindungen eingesetzt. Bevorzugt werden Allylether polyfunktioneller Alkohole eingesetzt. Besonders bevorzugt ist Tetraallyloxyethan. Ester der (Meth)Acrylsäure mit polyfunktionellen Alkoholen sind wegen ihrer Verseifbarkeit weniger geeignet.

Vorzugsweise werden nach dem erfindungsgemäßen Verfahren
30–70 Gewichtsteile Acrylamid,
70–30 Gewichtsteile Acrylsäure und/oder Methacrylsäure,
0–20 Gewichtsteile von copolymerisierbaren Monomeren der Formel III bzw. IX copolymerisiert.

Wenn ein Comonomer der Formel III eingesetzt wird, in dem X eine Alkoxycarbonylgruppe (–COOR) ist mit 6 bis 20 C-Atomen, so ist es besonders bevorzugt, dieses in einer Menge von höchstens 20 bis 6 Gew.% einzusetzen, wobei es besonders zweckmäßig ist, wenn die Höchstmenge (Höchstmenge III) dieses Monomers so bemessen wird, daß das Produkt
Höchstmenge III · C-Atom-Zahl der –COOR-Gruppe $\simeq$ 120 ist.

Acrylsäure und/oder Methacrylsäure können jede für sich oder im beliebigen Mischungsverhältnis bei der erfindungsgemäßen Copolymerisation eingesetzt werden. Vorzugsweise wird Methacrylsäure jedoch höchstens mit bis zu 30 Gew.% der Gesamtmenge des Säuregemischs eingesetzt. Besonders bevorzugt sind solche erfindungsgemäß hergestellten Copolymerisate, die keine Methacrylsäure-Bausteine enthalten.

Nach dem erfindungsgemäßen Verfahren kann in die Copolymerisate nicht nur eine einzelne Monomer-Spezies, sondern mehrere verschiedene Spezies der Formel III einpolymerisiert werden. Die oben angegebenen Mengenanteile beziehen sich dann auf die Summe der Monomerbestandteile der Formel III. Normalerweise werden nicht mehr als drei verschiedene, vorzugsweise eine, Verbindung der Formel III einpolymerisiert.

Im Hinblick auf die anwendungstechnischen Eigenschaften bei der Verwendung der Verdicker im Textildruck sowie mit Rücksicht auf den Preis der Produkte ist es bevorzugt, höchstens 20% copolymerisierbare Monomere der Formel III bzw. IX, insbesondere aber keine solchen copolymerisierbaren Monomeren einzubauen.

Die Eigenschaften der nach dem erfindungsgemäßen Verfahren erhaltenen Produkte hängen zu einem gewissen Ausmaß vom Wassergehalt des eingesetzten Lösungsmittels ab. Es ist daher bei der Herstellung spezieller Produkte zweckmäßig, in Gegenwart von bis zu 10 Gew.%, vorzugsweise bis zu 5 Gew.% Wasser, bezogen auf das Gewicht des Lösungsmittels, zu arbeiten.

Bei Wassergehalten über 10% besteht, besonders bei hohem (Meth)Acrylsäureanteil, die Gefahr der Verklumpung des Reaktionsgemisches. Weiterhin richtet sich der optimale Wassergehalt nach dem erwünschten Einsatzgebiet des Verdickungsmittels. Sollen besonders elektrolytstabile Produkte als Verdicker für den Textildruck erhalten werden, so wird bevorzugt kein Wasser oder ein Wassergehalt bis zu 1 Gew.% eingesetzt. In diesem Fall ist es häufig zweckmäßig, nach Abschluß der Polymerisation aber vor der Neutralisation dem Ansatz bis zu 5 Gew.% Wasser, bezogen auf die Menge des Lösungsmittels, zuzusetzen. Wird hingegen ein Verdickungsmittel für kosmetische Zwecke gesucht, bei dem ein besonders klares nicht körniges Gel erwünscht ist, so werden dem Alkohol vorzugsweise 2–5 Gew.% Wasser zugesetzt.

Die Menge des einzusetzenden Lösungsmittels richtet sich nach der Art des angestrebten Copolymers, weiterhin auch nach Art und Menge der vorgelegten Monomeren sowie nach dem Wassergehalt. In der Regel werden pro 100 g Gesamtmonomere höchstens 500 g, mindestens jedoch 200 g Lösungsmittel eingesetzt. Die bevorzugte Einsatzmenge liegt zwischen 480 g und 240 g pro 100 g Gesamtmonomeren.

Die Polymerisation wird in der Regel in einer Schutzgasatmosphäre, vorzugsweise unter Stickstoff, ausgeführt. Die Polymerisationstemperatur liegt zwischen 20° und 120 °C, vorzugsweise zwischen 30 und 90 °C.

Zur Auslösung der Polymerisation können energiereiche elektromagnetische Strahlen oder die üblichen chemischen Polymerisationsinitiatoren herangezogen werden, z. B. organische Peroxide, wie Benzolperoxid, tert. Butyl-hydroperoxid, Methylethyl-keton-peroxid, Cumol-hydroperoxid, Tert.-butylperoxy-3,5,5-trimethylhexanoat, Dicyclohexylperoxidicarbonat, Bis(4-t-butylcyclohexyl)-peroxidicarbonat, Azoverbindungen wie Azodi-iso-butyronitril oder 2'-Azo-bis-(2-amidinopropan)-dihydrochlorid

$$HN{=}\underset{\displaystyle NH_2}{\underset{|}{C}}{-}C(CH_3)_2{-}N{=}N{-}C(CH_3)_2{-}\underset{\displaystyle NH_2}{\underset{|}{C}}{=}NH \cdot 2\ HCl$$

sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren enthalten, wie z. B. Mannichaddukte aus Sulfinsäure, Aldehyden und Amino-Verbindungen, wie sie in der DE-PS-1 301 566 beschrieben sind. Pro 100 g Gesamtmonomeren werden in der Regel 0,03 bis 2 g des Polymerisationsinitiators eingesetzt.

Die Polymerisationsdauer beträgt in der Regel 1 bis 10 h; sie hängt in bekannter Weise von Polymerisationstemperatur und Initiatormenge ab.

Die Vielfältigkeit des Verfahrens gestattet es, jeweils angestrebte Produktionseigenschaften wie Viskositätsergiebigkeit, Elektrolytstabilität, Homogenität der wäßrigen Lösungen exakt einzustellen.

Darüber hinaus können noch sicherheitstechnische Aspekte, wie eine zuverlässige Beherrschbarkeit, aber auch wirtschaftliche Aspekte, wie

eine hohe Raum-Zeit-Ausbeute, mit den obigen Erfordernissen kombiniert werden.

Die Menge der vorgelegten Monomeren richtet sich nach dem Anforderungspektrum des Polymers. Zur Erzielung einer hohen Elektrolytstabilität werden vorzugsweise 10–50 Gew.% der Monomeren I bis III in 10 bis 75 Gew.% des Lösungsmittels vorgelegt. Wird darüber hinaus eine hohe Raum-Zeit-Ausbeute gewünscht in der Art, daß hochkonzentrierte Polymerdispersionen erhalten werden sollen durch Polymerisation von Monomerlösungen mit über 20 Gew.%, insbesondere über 25 Gew.%, Monomeren, so werden vorzugsweise 10 bis 20 Gew.% der Gesamtmenge der Monomeren in 10 bis 50 Gew.% Lösungsmittel vorgelegt. Wird bei Polymerisation von Monomerenlösungen über 25 Gew.% zusätzlich noch Wert auf eine hohe Viskositätsergiebigkeit gelegt, so werden vorzugsweise 10 bis 20 Gew.% der Gesamtmenge der Monomeren in vorzugsweise 10 bis 20 Gew.% der Gesamtmenge des Lösungsmittels vorgelegt. Wird Wert auf einen möglichst geringen Abfall nach Elektrolytzusatz gelegt, so werden bei Polymerisation von Monomerlösungen mit über 25 Gew.% Monomeren 10 bis 20 Gew.% der Gesamtmenge der Monomeren vorgelegt in vorzugsweise 20 bis 50 Gew.% des Lösungsmittels und die zuzuführende restliche Monomerlösung entsprechend aufkondensiert. Dieses Verfahren hat zudem noch den bedeutenden Vorteil der Möglichkeit zur besseren Wärmeabfuhr.

Die Eigenschaften der erfindungsgemäß hergestellten Copolymerisate können zusätzlich dadurch gesteuert werden, daß die Copolymerisation in Gegenwart von bis zu 10 Gew.% Wasser, bezogen auf das Gesamtgewicht des Lösungsmittels, durchgeführt wird.

Der Wassergehalt des Lösungsmittels richtet sich wiederum nach dem Anwendungsspektrum. Zur Erzielung einer hohen Viskositätsergiebigkeit in elektrolytfreiem Wasser werden vorzugsweise 1–10 Gew.% Wasser, vorzugsweise 1–5 Gew.% Wasser, dem Lösungsmittel zugesetzt. Die Aufteilung des Wassergehaltes auf Vorlage und Zulauf erfolgt vorzugsweise in der Art, daß der zur vorgelegten Monomerenmenge aliquote Teil Wasser vorgelegt, d. h. 10–50 Gew.% der Gesamtmenge an Wasser vorgelegt werden.

Ist ein besonders klares nicht körniges Gel erwünscht, so werden vorzugsweise 2–5 Gew.% Wasser dem Lösungsmittel zugesetzt. Die Aufteilung erfolgt vorzugsweise in der Art, daß der zur vorgelegten Monomerenmenge aliquote Teil Wasser vorgelegt wird. Wird ein besonders elektrolytstabiles Polymer gewünscht, so wird vorzugsweise kein oder ein Wassergehalt bis zu 1 Gew.% bezogen auf Lösungsmittel eingesetzt. Vorzugsweise werden dann 1 bis 5 Gew.% Wasser erst nach der Polymerisation aber vor der Neutralisation dem Gesamtgemisch zugesetzt.

Sollen Elektrolytstabilität, Klarheit, geringe Körnigkeit und Ergiebigkeit der Gele angestrebt werden, so werden vorzugsweise 1–5 Gew.% Wasser zugesetzt; die Aufteilung des Wassers erfolgt in der Art, daß vorzugsweise der zur vorgelegten Monomerenmenge aliquote Teil Wasser vorgelegt wird, besonders bevorzugt werden 10–40 Gew.% des Wassers vorgelegt.

Die Zusammensetzung der Monomeren im Zulauf und Vorlage kann in weiten Grenzen variiert werden. Hinsichtlich Polymerisationsverlauf ist es bevorzugt, daß mindestens 20 Gew.% der in der Vorlage vorgelegten Monomere Acrylamid ist. Bei niedrigeren Acrylamidanteilen in der Vorlage besteht die Möglichkeit der Verklumpung des Reaktionsgemisches. Vorzugsweise weicht die Zusammensetzung des Monomerengemisches in der Vorlage nicht von der des Zulaufs ab.

Die Aufteilung des Vernetzers auf Vorlage und Zulauf ist weitgehend in der Art variabel, daß 10–100 Gew.% des Vernetzers, bevorzugt jedoch 10–50 Gew.%, besonders bevorzugt der zu den vorgelegten Monomeren aliquote Teil des Vernetzers, vorgelegt werden können. Werden 100 Gew.% Vernetzer vorgelegt, so werden Polymere mit hoher Viskositätsergiebigkeit und geringer Elektrolytstabilität erhalten. Werden 100 Gew.% des Vernetzers zudosiert, so werden wenig viskositätsergiebige Polymere erhalten.

Bei Zudosierung von hochkonzentrierten Monomerenlösungen erfolgt aus Sicherheitsgründen bevorzugt eine getrennte Zuführung des Initiators, bevorzugt als Lösung. Die Zuführung erfolgt in der Art, daß der jeweils aliquote Teil Initiatorlösung und Monomerlösung in gleicher Zeiteinheit zudosiert werden. Bei weiterer Aufteilung der Bestandteile des Zulaufs wird bevorzugt so zudosiert, daß jeweils den zugeführten Monomeren aliquote Teile der weiteren Bestandteile des Zulaufs in gleicher Zeiteinheit zugefügt werden.

Die während der Polymerisation zugeführten Bestandteile werden in 1 bis 10 h, bevorzugt in 1–4 h, besonders bevorzugt in 2–4 h, zugefügt. Längere Zulaufzeiten führen im allgemeinen zu höheren Viskositätsergiebigkeiten. Weiterhin ist es bevorzugt, die Zulaufgeschwindigkeit so zu wählen, daß eine wirtschaftlich sinnvolle Polymerisationsgeschwindigkeit sichergestellt wird.

Die Polymerisationstemperatur liegt zwischen 20 und 120 °C, vorzugsweise 30–90 °C. Sie wird besonders bevorzugt so gewählt, daß die verwendeten Initiatoren eine Halbwertzeit von 1–100 h, vorzugsweise 1–50 h, haben.

Die Initiatordosierung erfolgt in üblicher Weise so, daß der zu den vorgelegten Monomeren aliquote Teil Initiator vorzugsweise vorgelegt wird. In der Regel werden so höhere Viskositätsergiebigkeiten erzielt. Aus verfahrenstechnischen Gründen, etwa um eine genügend hohe Polymerisationsgeschwindigkeit sicherzustellen, kann auch mehr Initiator, bis zu 100 Gew.%, vorgelegt werden. Die so erhaltenen Produkte zeichnen sich durch einen geringeren Viskositätsabfall nach Elektrolytzusatz bei geringerer Viskositätsergiebigkeit aus.

Bei Durchführung der Polymerisation nach dem erfindungsgemäßen Verfahren bereitet die Wärmeabfuhr auch bei hohen Monomerenkonzentrationen im Lösungsmittel keine Schwierigkeiten, und man erhält gut rührbare Polymerisatpasten.

Die Polymerisatpasten sind ohne weiteres für die anschließende Aufarbeitung geeignet. Sie können mit Alkalihydroxiden direkt in die Salze überführt werden, wenn nicht bei der Polymerisation von vornherein die Salze der Acrylsäure oder Methacrylsäure eingesetzt wurden. Die Ammoniumsalze werden durch Einleiten von $NH_3$(g) oder Umsetzung mit wäßrigem Ammoniak erhalten. Es ist sogar möglich, feste Alkalihydroxide einzusetzen, dabei ist es besonders vorteilhaft, wenn das Lösungsmittel bis zu 5% Wasser enthält. Es ist auch möglich, das Polymerisat in Salz- oder «saurer» Form durch Absaugen oder Abdestillieren des Lösungsmittels zu isolieren.

Wird das Polymerisat für den Textildruck eingesetzt, so wird es in der Regel nicht zwischenisoliert. Das Polymerisat wird in der Salzform durch Abdestillieren des Lösungsmittels und Ersatz durch höhersiedende isoparaffinische Kohlenwasserstoffe direkt als Druckpaste formuliert. Bei Auswahl geeigneter W/O-Emulgatoren oder Schutzkolloide erhält man stabile nicht absetzende Dispersionen des Polymers in Kohlenwasserstoffen. Geeignete isoparaffinische Kohlenwasserstoffe sind unter den Namen ®Esso Varsol, ®Esso Exsol oder ®Esso Isopar bekannt.

Geeignete W/O-Emulgatoren sind in der Regel Sorbitanfettsäureester, die unter den Handelsnamen ®Span und ®Tween angeboten werden. Darüberhinaus können auch noch Fettsäureester, ethoxilierte Fettsäureester, Fettalkohole und ethoxilierte Fettalkohole sowie Blockpolymerisate vom Typ Ethylenoxid-Propylenoxid oder Ethylenoxid-Butylenoxid eingesetzt werden.

Es ist überraschend und nicht vorhersehbar, daß eine verzögerte Zugabe von Monomeren und gegebenenfalls weiteren Bestandteilen des Polymerisationsansatzes während der Polymerisation nach dem erfindungsgemäßen Verfahren es gestattet, auf einfache und praktisch kostenneutrale Weise das Eigenschaftsspektrum der erhaltenen Polymerisate nach Bedarf weitgehend zu variieren und insbesondere zu einer drastisch höheren Elektrolytstabilität führt. Es gelingt nicht, durch zwei hintereinandergeschaltete Eintopfverfahren, in denen erst ein hoch vernetztes und anschließend ein niedriger vernetztes Polymerisat oder umgekehrt hergestellt wird, vergleichbar gute Produkte zu erhalten. Weiterhin ist der Effekt, den der Wassergehalt des Lösungsmittels auf die Elektrolytstabilität ausübt, völlig überraschend und nicht vorhersehbar.

Auch die Art der Initiatordosierung hat auf Molekulargewicht und die Elektrolytstabilität einen Einfluß, der völlig überraschend und nicht vorhersehbar ist.

Für den Textildruck beinhaltet die Anwendung der Erfindung besondere Vorteile. Neben dem erwähnten Vorteil, daß das Polymer nicht zwischenisoliert werden muß, läßt sich weiterhin der für den Druck erforderliche Viskositätsbereich exakt bei der Polymerisation einstellen. Druckpasten für den textilen Pigmentdruck sollen auf einen Viskositätsbereich von 60–80 dPas eingestellt werden. Beim Einrühren des Verdickungsmittels in Wasser muß aber ein höherer Viskositätswert in Kauf genommen werden, der umso höher liegt, je größer die Elektrolytempfindlichkeit des als Verdicker eingesetzten Copolymerisats ist, und der dann bei Zusatz der üblichen elektrolythaltigen Bestandteile der Druckfarbe, wie z. B. Binder, Fixierer und evtl. Säurespender ($(NH_4)_2SO_4$ oder $(NH_4)_3PO_4$) auf den gewünschten Viskositätswert der Druckpaste abfällt.

Dabei ist ein möglichst niedriger Ausgangswert der Viskosität, der durch den Elektrolytgehalt der weiteren Bestandteile der Druckfarbe auf 60–80 dPas absinkt, erwünscht. Ein niedriger Ausgangswert erleichtert das Einarbeiten der weiteren Bestandteile der Druckfarbe, es können so leichter homogene Farben hergestellt werden. Unbrauchbar sind Verdickungsmittel, deren Ausgangsviskosität über 160 dPas liegt, da sich dann die übrigen Bestandteile nur noch schwer einarbeiten lassen. Im großtechnischen Maßstab ist eine Einarbeitung dann nahezu unmöglich. Durch das erfindungsgemäße Verfahren lassen sich hervorragende Verdickungsmittel für den textilen Pigmentdruck herstellen, die nur einen geringen Viskositätsabfall durch Elektrolyteinfluß aufweisen. Für den textilen Pigmentdruck geeignete Produkte enthalten bevorzugt 30–70 Gew.% Acrylamid und 70–30 Gew.% Acrylsäure sowie 0–2 Gew.% Vernetzer.

Selbstverständlich is es auch möglich, das nach dem erfindungsgemäßen Verfahren erhaltene Copolymerisat ohne vorherige Neutralisation, d. h. in «saurer» Form zu isolieren.

Die so hergestellten erfindungsgemäßen Copolymerisate können vorzugsweise zur Viskositätserhöhung und Stabilisierung von kosmetischen, pharmazeutischen und technischen Präparaten eingesetzt werden. Beispielsweise lassen sich die erfindungsgemäßen Substanzen nach Neutralisation mit Alkalien zur Stabilisierung von wasser- und/oder lösungsmittelhaltigen Emulsionen, Suspensionen oder Lösungen verwenden. Durch Zusatz dieser viskositätserhöhenden und stabilisierenden Copolymerisate, vorwiegend in neutralisierter Form, wird in den entsprechenden kosmetischen, pharmazeutischen oder technischen Präparaten eine wesentliche Verbesserung der physikalischen Stabilität in Abhängigkeit von der Lagerzeit und Temperatur erzielt.

Außerdem beeinflussen die erfindungsgemäßen Konsistenzgeber auch das anwendungstechnische Verhalten günstig. Beispielsweise wird bei technischen Präparaten durch die erzielte hohe Viskosität eine bessere Haftung auf dem zu behandelnden Untergrund erreicht. Bei Einsatz in pharmazeutischen oder kosmetischen Präparaten bewirkt die hohe Viskosität eine günstigere Verteilbarkeit auf Haar- und Hautoberflächen, sowie eine bessere Haftung der Präparate auf der Körperoberfläche.

Im Gegensatz zu den gängigen Konsistenzgebern, wie Stärke, Gelatine, Agar-Agar oder Traganth, sind die wäßrigen Gele auf Basis der erfindungsgemäßen Copolymerisate auch weniger gegen Mikroorganismen anfällig. Ein weiterer Vorteil ist das günstige rheologische Verhalten in Abhängigkeit von der Temperatur. Das heißt, auch bei höheren Lagertemperaturen, wie z. B. +45 °C sinkt die Viskosität im Vergleich zur Raumtemperatur von ca. +20 °C nicht wesentlich ab.

In Abhängigkeit von der eingesetzten Menge an Copolymerisat bzw. dem verwendeten Neutralisationsmittel können beispielsweise mit Wasser klare und vollkommen homogene Lösungen, die keine Mikrogele aufweisen, erhalten werden. Ein weiterer Vorteil dieser Lösungen ist ihr günstiges rheologisches Verhalten, d. h. die entsprechenden Präparate ziehen «keine Fäden». Dies ist vor allem ein wesentlicher Vorteil bei der praktischen Anwendung der pharmazeutischen, kosmetischen und technischen Präparate.

Die in den nachstehenden Beispielen aufgeführten Formulierungen zur Herstellung von technischen, kosmetischen und pharmazeutischen Präparaten enthalten bevorzugt Copolymerisate auf Basis 30 bis 70 Gew.% Acrylamid, 70 bis 30 Gew.% Acrylsäure un d0,3 bis 2,5 Gew.% Vernetzer.

Weiterhin können bevorzugte, erfindungsgemäß hergestellte Verdickungsmittel für kosmetische Zubereitungen bis zu 10% Vinylphosphonsäure oder Vinylphosphonsäureanhydrid enthalten.

Beispiel 1:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 440 ml tert.Butanol und 15 ml entionisiertes Wasser vorgelegt. Unter Rühren werden darin 49,7 g Acrylamid und 21,3 g Acrylsäure gelöst, und dann wird dieser Lösung 0,75 g Tetraallyloxyethan zugesetzt.

Im Tropftrichter werden 440 ml tert.Butanol und 15 ml entionisiertes Wasser vorgelegt, unter Rühren darin 49,7 g Acrylamid und 21,3 g Acrylsäure gelöst und der Lösung 0,75 g Tetraallyloxyethan und 1 g Azodiisobutyronitril zugesetzt.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert.

Zurück bleiben 415 g (~ 100% der Theorie) einer gießbaren, nicht absetzenden ca. 37%igen Dispersion des Polymers in dem Kohlenwasserstoffgemisch.

Zur Prüfung der Verdickungswirkung des erhaltenen Polymerisats werden 40 g der 37%igen Dispersion auf 1000 g mit entmineralisiertem Wasser verdünnt und gerührt. Innerhalb einer Minute erhält man durch Quellung des Polymers eine weiße viskose Masse, die bis zur vollständigen Auflösung maximal 5 Minuten gerührt wird. Die Viskosität der Masse wird mit einem Rotationsviskosimeter, dem «Haake Viskotester» zu 130 dPas bestimmt. 850 bis 920 g der zuvor hergestellten Masse werden mit 80 bis 150 g eines handelsüblichen Binders, z. B. Imperon® Binder MTB (Imperon® ist ein eingetragenes Warenzeichen der Hoechst AG, Frankfurt am Main), unter Rühren versetzt. Durch den Elektrolytgehalt des Binders sinkt die Viskosität des Systems auf 80 dPas ab. Den gleichen Abfall beobachtet man, wenn 1000 g der hergestellten Masse mit einer Lösung von 1,1 g Ammoniumsulfat in 2,2 g entmineralisiertem $H_2O$ (einem häufig im Pigmentdruck eingesetzten Säurespender) oder mit einer Lösung von 1,1 g Natriumchlorid in 5 ml entmineralisiertem Wasser unter Rühren versetzt werden.

Eine einfache Möglichkeit, charakteristische Daten für die Verdickungswirkung der hergestellten Copolymerisatdispersionen zu erhalten, besteht darin, die Viskositäten in reinem Wasser und in einer ca. 0,11gew.%igen Ammonsulfat- oder Natriumchloridlösung bei mehreren verschiedenen Polymerkonzentrationen zu bestimmen.

Um direkt vergleichbare Zahlenwerte für die Verdickungswirkung der hergestellten Copoylmerisate zu erhalten, kann dann z. B. angegeben werden, wieviel Gew.% der 37%igen Dispersion erforderlich sind, um in einer der oben angegebenen Elektrolytlösungen eine bestimmte Viskosität von z. B. 70 dPas zu erhalten und wie hoch bei gleicher Dispersionsmenge die Viskosität in reinem Wasser ansteigt. Im vorliegenden Beispiel müssen zur Einstellung einer Viskosität von 70 dPas der 0,11gew.%igen Ammonsulfatlösung 2,86 Gew.% (2,9 g Dispersion + 97,1 g Elektrolytlösung) der 37%igen Polymerisatdispersion zugesetzt werden.

Der gleiche Polymerzusatz in reinem Wasser führt dann zu einer Viskosität von 119 dPas.

In den folgenden Ausführungs- und Vergleichsbeispielen sowie in den Tabellenbeispielen wird folgendes Datenschema zur Charakterisierung verwendet:

| Viskosität der Elektrolytlösung in dPas | Zur Einstellung dieser Viskosität erforderliche Konzentration der 37%igen Polymerlösung in der Elektrolytlösung | Viskosität, die sich bei Einstellung der gleichen Polymerkonzentration in reinem Wasser ergibt in dPas |
|---|---|---|

Für obiges Beispiel hat dieses Datenschema die Werte 70/2,86–119.

Das Ausführungsbeispiel 1 wird wiederholt mit der Abänderung, daß man das Neutralisieren mit Ammoniak unterläßt und statt dessen in den Ansatz 23,6 g festes, pulverisiertes Natriumhydroxid einrührt. Man setzt das Rühren noch fort bis zur vollständigen Auflösung des Natriumhydroxids und setzt dann die Aufarbeitung wie oben beschrieben fort. Man erhält 420 g der erfindungsgemäßen Copolymerisatdispersion Nr. 14 der Tabelle 1 mit den Kenndaten 70/2,47–123.

Beispiel 2:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 440 ml tert.Butanol vorgelegt. Unter Rühren werden darin 49,7 g Acrylamid und 21,3 g Acrylsäure gelöst, und dann wird dieser Lösung 0,75 g Tetrallyloxyethan zugesetzt.

Im Tropftrichter werden 440 ml tert.Butanol vorgelegt, unter Rühren darin 49,7 g Acrylamid und 21,3 g Acrylsäure gelöst und der Lösung 0,75 g Tetraallyloxyethan und 1 g Azodiisobutyronitril zugesetzt.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,3–123.

Beispiel 3:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 400 ml tert.Butanol vorgelegt. Unter Rühren werden darin 21,3 g Acrylamid und 49,7 g Acrylsäure gelöst, und dann wird dieser Lösung 1,2 g Tetraallyloxyethan zugesetzt.

Im Tropftrichter werden 440 ml tert.Butanol vorgelegt, unter Rühren darin 21,3 g Acrylamid und 49,7 g Acrylsäure gelöst und der Lösung 1,2 g Tetraallyloxyethan und 1 g tert.Butylperoxy-3,5,5-trimethylhexanoat zugesetzt.

Das Reaktionsgefäß wird unter Rühren und Einleiten eines schwachen Stickstoffstromes auf 80 °C erwärmt und bei dieser Temperatur 1 g tert.-Butylperoxy-3,5,5-trimethylhexanoat, gelöst in 40 ml tert.Butanol, zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 2 °C angestiegen ist, wird die Monomerlösung aus dem Tropftrichter innerhalb von 2 Stunden gleichmäßig zugetropft. Die Polymerisationstemperatur steigt sofort auf 82 °C an. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das

Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/3–108.

Beispiel 4:

In einem Polymerisationsgefäß, ausgestattet mit Rührer, Rückflußkühler, Thermometer, Gaseinleitungsrohr, zwei Tropftrichtern mit eigenem Rührer und elektrisch beheiztem Wasserbad werden 90 ml tert.Butanol vorgelegt. Unter Rühren werden darin 9,9 g Acrylamid und 4,2 g Acrylsäure gelöst, und dann wird dieser Lösung 0,15 g Tetraallyloxyethan und 0,5 g Bis-(4-tert.butyl-cyclohexyl)-peroxidicarbonat zugesetzt.

Im ersten Tropftrichter werden 450 ml tert.Butanol vorgelegt, unter Rühren darin 89,5 g Acrylamid und 38,4 g Acrylsäure gelöst und der Lösung 1,35 g Tetraallyloxyethan zugesetzt. Der zweite Tropftrichter wird mit einer Lösung von 0,5 g Bis-(4-tert.butyl-cyclohexyl)-peroxidicarbonat in 40 ml tert.Butanol beschickt.

Das Reaktionsgefäß wird unter Rühren und Einleiten eines schwachen Stickstoffstromes auf 48 °C erwärmt. Nach etwa 10 bis 30 Minuten setzt die Polymerisation mit einem deutlichen Temperaturanstieg ein. Sobald die Temperatur im Reaktionsgefäß um ca. 3 °C angestiegen ist, wird die Monomerlösung aus dem ersten Tropftrichter und die Initiatorlösung aus dem zweiten Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,2–115.

Wird nur 0,1 g Initiator vorgelegt und 0,9 g nach Beginn der Polymerisation zudosiert, so erhält man ein erfindungsgemäßes Copolymerisat mit den Kenndaten: 70/2,0–130.

Beispiel 5:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 176 ml tert.Butanol und 6 ml entionisiertes Wasser vorgelegt. Unter Rühren werden darin 19,9 g Acrylamid und 8,5 g Acrylsäure gelöst, und dann wird dieser Lösung 0,3 g Tetraallyloxyethan zugesetzt.

Im Tropftrichter werden 704 ml tert.Butanol und 24 ml entionisiertes Wasser vorgelegt, unter Rühren darin 79,5 g Acrylamid und 34,1 g Acrylsäure gelöst und der Lösung 1,2 g Tetraallyloxyethan und 1,6 g Bis-(4-tert.-butyl-cyclohexyl)-peroxidicarbonat zugesetzt.

Das Reaktionsgefäß wird unter Rühren und Einleiten eines schwachen Stickstoffstromes auf 48 °C erwärmt und bei dieser Temperatur 0,4 g Bis-(4-tert.butyl-cyclohexyl)-peroxidicarbonat zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,4–110.

Durch Variation der obigen Versuchsbedingungen in der in Tabelle 1 angegebenen Weise lassen sich die ebenfalls sehr vorteilhaften erfindungsgemäßen Copolymerisatdispersionen 15 und 16 herstellen.

Beispiel 6:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 440 ml tert.Butanol und 15 ml entionisiertes Wasser vorgelegt. Unter Rühren werden darin 35,5 g Acrylamid, 21,3 g Acrylsäure und 14,2 g 2-Acrylamido-2-methylpropansulfonsäure gelöst, und dann wird dieser Lösung 0,75 g Tetraallyloxyethan zugesetzt.

Im Tropftrichter werden 440 ml tert.Butanol und 15 ml entionisiertes Wasser vorgelegt, unter Rühren darin 35,5 g Acrylamid, 21,3 g Acrylsäure und 14,2 g 2-Acrylamido-2-methylpropansulfonsäure gelöst und der Lösung 0,75 g Tetraallyloxyethan und 1,0 g Azodiisobutyronitril zugesetzt.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rücflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,51–122.

Beispiel 7:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 88 ml tert.Butanol und 3 ml entionisiertes Wasser vorgelegt. Unter Rühren werden darin 9,9 g Acrylamid und 4,3 g Acrylsäure gelöst, und dann wird dieser Lösung 0,15 g Tetraallyloxyethan zugesetzt.

Im Tropftrichter werden 792 ml tert.Butanol und 27 ml entionisiertes Wasser vorgelegt, unter Rühren darin 89,5 g Acrylamid und 38,3 g Acrylsäure gelöst und der Lösung 1,35 g Tetraallyloxyethan und 1,8 g Azodiisobutyronitril zugesetzt.

Das Reaktionsgefäß wird unter Rühren und Einleiten eines schwachen Stickstoffstromes auf 58 °C erwärmt und bei dieser Temperatur 0,2 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/3,1–118.

Durch Variation der Versuchsbedingungen in der in Tabelle 1 angegebenen Weise erhält man die Copolymerisate Nr. 17 und 18.

Beispiel 8:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 400 ml tert.Butanol und 10 ml entionisiertes Wasser vorgelegt. Unter Rühren werden darin 42,6 g Acrylamid und 28,4 g Acrylsäure gelöst, und dann wird dieser Lösung 0,6 g Tetraallyloxyethan zugesetzt.

Im Tropftrichter werden 440 ml tert.Butanol und 10 ml entionisiertes Wasser vorgelegt, unter Rühren darin 42,6 g Acrylamid und 28,4 g Acrylsäure

gelöst und der Lösung 0,6 g Tetraallyloxyethan und 1 g tert.Butylperoxy-3,5,5-trimethylhexanoat zugesetzt.

Das Reaktionsgefäß wird unter Rühren und Einleiten eines schwachen Stickstoffstromes auf 80 °C erwärmt und bei dieser Temperatur 1 g tert.-Butylperoxy-3,5,5-trimethylhexanoat, gelöst in 40 ml tert.Butanol, zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 2 °C angestiegen ist, wird die Monomerlösung aus dem Tropftrichter innerhalb von 2 Stunden gleichmäßig zugetropft. Die Polymerisationstemperatur steigt sofort auf 82 °C an, das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,62–127.

Durch Variation der obigen Versuchsbedingungen in der in Tabelle 1 angegebenen Weise läßt sich die ebenfalls sehr vorteilhafte erfindungsgemäße Copolymerisatdispersion 19 herstellen.

Beispiel 9:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 440 ml tert.Butanol und 10 ml entionisiertes Wasser vorgelegt. Unter Rühren werden darin 35,5 g Acrylamid und 35,5 g Acrylsäure gelöst, und dann wird dieser Lösung 1 g Tetraallyloxyethan zugesetzt.

Im Tropftrichter werden 440 ml tert.Butanol und 10 ml entionisiertes Wasser vorgelegt, unter Rühren darin 35,5 g Acrylamid und 35,5 g Acrylsäure gelöst und der Lösung 1 g Tetraallyloxyethan und 1 g Azodiisobutyronitril zugesetzt.

Das Reaktionsgefäß wird unter Rühren und Einleiten eines schwachen Stickstoffstromes auf 40 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 60 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,9–135.

Beispiel 10:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 68 ml tert.Butanol und 3 ml entionisiertes Wasser vorgelegt. Unter Rühren werden darin 9,94 g Acrylamid und 4,26 g Acrylsäure gelöst, und dann wird dieser Lösung 0,15 g Tetraallyloxyethan zugesetzt.

Im Tropftrichter werden 540 ml tert.Butanol und 27 ml entionisiertes Wasser vorgelegt, unter Rühren darin 89,5 g Acrylamid und 38,34 g Acrylsäure gelöst und der Lösung 1,35 g Tetraallyloxyethan zugesetzt. In einem zweiten Tropftrichter werden 1,8 g Bis-(4-tert.butylcyclohexyl)-peroxidicarbonat in 72 ml tert.Butanol gelöst.

Das Reaktionsgefäß wird unter Rühren und Einleiten eines schwachen Stickstoffstromes auf 48 °C erwärmt und bei dieser Temperatur 0,2 g Bis-(4-tert.butyl-cyclohexyl)-peroxidicarbonat zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung und die Initiatorlösung aus den Tropftrichtern innerhalb von 2 Stunden gleichmäßig zugetropft. Die Polymerisationstemperatur steigt innerhalb von 1 Stunde auf 55 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reatkionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,4–140.

Beispiel 11:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 630 ml tert.Butanol und 15 ml entionisiertes Wasser vorgelegt. Unter Rühren werden darin 49,7 g Acrylamid und 21,3 g Acrylsäure gelöst, und dann wird dieser Lösung 0,6 g Tetraallyloxyethan zugesetzt.

Im Tropftrichter werden 150 ml tert.Butanol und 15 ml entionisiertes Wasser vorgelegt, unter Rühren darin 49,7 g Acrylamid und 21,3 g Acrylsäure gelöst und der Lösung 1,4 g Tetraallyloxyethan zugesetzt. 1 g Azodiisobutyronitril wird in einem zweiten Tropftrichter in 100 ml tert.Butanol gelöst.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung und die Initiatorlösung aus den Tropftrichtern innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/2,9–130.

Durch Variation der obigen Veruschsbedingungen in der in Tabelle 1 angegebenen Weise lassen sich die ebenfalls sehr vorteilhaften erfindungsgemäßen Copolymerisatdispersionen 20 und 21 herstellen.

Beispiel 12:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 630 ml tert.Butanol und 15 ml entionisiertes Wasser vorgelegt. Unter Rühren werden darin 49,7 g Acrylamid und 21,3 g Acrylsäure gelöst, und dann wird dieser Lösung 1 g Tetraallyloxyethan als Vernetzer und 0,04 g Dodecylmercaptan als Regler zugesetzt.

Im Tropftrichter werden 150 ml tert.Butanol und 15 ml entionisiertes Wasser vorgelegt, unter Rühren darin 49,7 g Acrylamid und 21,3 g Acrylsäure gelöst und der Lösung 1 g Tetraallyloxyethan zugesetzt. In einem zweiten Tropftrichter wird 1 g Azodiisobutyronitril in 100 ml tert.Butanol gelöst.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung und die Initiatorlösung aus den Tropftrichtern innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Der Rückflußkühler wird durch eine Destillationsbrücke ersetzt, durch den Tropftrichter wird die Lösung von 7,0 g eines Sorbitanfettsäureesters, 9,8 g eines ethoxilierten Nonylphenols sowie 2,8 g eines Ethylenoxid-Propylenoxid-Blockpolymerisats in 239,0 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben 415 g (~100% der Theorie) einer gießbaren, nicht absetzenden ca. 37%igen Dispersion des Polymers in dem Kohlenwasserstoffgemisch.

Kenndaten: 70/3,2–115.

Durch Aufteilung der Reglersubstanz auf Vorlage und Zulauf läßt sich die ebenfalls sehr vorteilhafte erfindungsgemäße Copolymerisatdispersion 22 der Tabelle 1 herstellen.

Beispiel 13:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 440 ml tert.Butanol vorgelegt. Unter Rühren werden darin 49,7 g Acrylamid und 21,3 g Acrylsäure gelöst, und dann wird dieser Lösung 1,0 g Tetraallyloxyethan zugesetzt. Anschließend werden 20 ml 25gew.%ige wäßrige Ammoniaklösung zugefügt.

Im Tropftrichter werden 440 ml tert.Butanol vorgelegt, unter Rühren darin 49,7 g Acrylamid und 21,3 g Acrylsäure gelöst und der Lösung 1,0 g Tetraallyloxyethan und 1 g Azodiisobutyronitril zugesetzt. Danach werden noch 20 ml 25gew.%ige wäßrige Ammoniaklösung zugefügt.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird das Polymerisationsgefäß mit einem Homogenisiergerät ausgerüstet und die Polymerisatdispersion durch mehrfaches Umpumpen über das Homogenisiergerät gut homogenisiert.

Zur Aufarbeitung und Abdestillation des tert. Butanols werden der Polymerisatpaste 7 g eines Sorbitanfettsäureesters, 9,8 g eines ethoxilierten Nonylphenols sowie 2,8 g eines Ethylenoxid-Propylenoxid-Blockpolymerisats in 360 g eines isoparaffinischen Kohlenwasserstoffgemisches vom Siedebereich 150 bis 350 °C gelöst zugetropft und anschließend das tert.Butanol abdestilliert. Zurück bleiben ca. 534 g (~100% der Theorie) einer gießbaren nicht absetzenden ca. 29%igen Dispersion des Polymers im Kohlenwasserstoffgemisch.

Kenndaten: (umgerechnet auf 37gew.%ige Dispersion) 70/2,49–136,5.

Durch Variation der Versuchsbedingungen gemäß den Angaben in der folgenden Tabelle 1 werden auch die erfindungsgemäßen Copolymerisate Nr. 23 bis 32 erhalten. In der Tabelle 1 werden die folgenden Abkürzungen verwendet:

A = Azodiisobutyronitril
B = Bis-(4-tert.butyl-cyclohexyl)-peroxidicarbonat
C = tert.Butylperoxy-3,5,5-trimethylhexanoat
R = Dodecylmercaptan (Regler)
TAE = Tetraallyloxyethan
TPTA = Trimethylolpropan-trimethacrylat
(a) = gelöst in 100 ml tert. Butanol
(b) = gelöst in 80 ml tert. Butanol
(c) = gelöst in 72 ml tert. Butanol

Tabelle 1

| Vorlage Nr. | AM (g) | AS (g) | Reg. (g) | TAE (g) | Initi. | Initi. (g) | t.BuOH (ml) | $H_2O$ (ml) | Temp. (°C) | Zulauf AM (g) | AS (g) | Reg. (g) | TAE (g) | Initi. | Initi. (g) | t.BuOH (ml) | $H_2O$ (ml) | Kenndaten |
|---|---|---|---|---|---|---|---|---|---|---|---|---|---|---|---|---|---|---|
| 14 | 49,7 | 21,3 | – | 0,75 | A | 1,0 | 630 | 15 | 50 | 49,7 | 21,3 | – | 0,75 | A | 1,0 | 150 | 15 | 70/2,47–123 |
| 15 | 9,94 | 4,26 | – | 0,15 | B | 0,2 | 88 | 3 | 51 | 89,5 | 38,3 | – | 1,35 | B | 1,8 | 792 | 27 | 70/2,6–108 |
| 16 | 19,9 | 8,5 | – | 0,3 | A | 0,4 | 176 | 6 | 58 | 79,5 | 34,1 | – | 1,2 | A | 1,6 | 704 | 24 | 70/2,4–100 |
| 17 | 29,8 | 12,8 | – | 0,45 | A | 0,4 | 264 | 9 | 56 | 69,6 | 29,8 | – | 1,05 | A | 1,4 | 616 | 21 | 70/2,7–139 |
| 18 | 59,6 | 25,6 | – | 0,9 | B | 1,0 | 528 | 18 | 48 | 39,8 | 17,0 | – | 0,6 | B | 0,8 | 352 | 12 | 70/3,2–99 |
| 19 | 49,7 | 21,3 | – | 0,43 | C | 1,0 | 400 | 15 | 80 | 49,7 | 21,3 | – | 0,43 | C | 1,0 | 440 | 15 | 70/1,7–123 |
| 20 | 49,7 | 21,3 | – | 0,7 | A | 1,0 | 630 | 15 | | 49,7 | 21,3 | – | 1,3 | A | 1,0(a) | 150 | 15 | 70/2,9–135 |
| 21 | 49,7 | 21,3 | – | 0,75 | A | 1,0 | 630 | 15 | 50 | 49,7 | 21,3 | – | 1,25 | A | 1,0(a) | 150 | 15 | 70/3,0–132 |
| 22 | 49,7 | 21,3 | R 0,02 | 1,0 | A | 1,0 | 650 | 15 | 50 | 49,7 | 21,3 | R 0,02 | 1,0 | A | 1,0(b) | 150 | 15 | 70/2,9–135 |
| 23 | 49,7 | 21,3 | – | 1,0 | A | 2,0 | 730 | 15 | 50 | 49,7 | 21,3 | – | 1,0 | – | – | 150 | 15 | 70/3,6–115 |
| 24 | 49,7 | 21,3 | R 0,01 | 1,0 | A | 1,0 | 650 | 15 | 50 | 49,7 | 21,3 | R 0,01 | 1,0 | A | 1,0(b) | 150 | 15 | 70/3,0–137 |
| 25 | 49,7 | 21,3 | – | 1,0 | A | 1,0 | 630 | 15 | 50 | 49,7 | 21,3 | R 0,04 | 1,0 | A | 1,0(a) | 150 | 15 | 70/3,0–126 |
| 26 | 49,7 | 21,3 | – | 0,5 | A | 1,0 | 630 | 15 | 50 | 49,7 | 21,3 | – | 1,5 | A | 1,0(a) | 150 | 15 | 70/3,3–123 |
| 27 | 42,6 | 28,4 | – | 0,75 | A | 1,0 | 630 | 15 | 50 | 56,8 | 14,2 | – | 1,25 | A | 1,0(a) | 150 | 15 | 70/3,1–138 |
| 28 | 28,4 | 42,6 | – | 0,6 | A | 1,0 | 630 | 15 | 50 | 71 | – | – | 1,4 | A | 1,0(a) | 150 | 15 | 70/2,9–140 |
| 29 | 28,4 | 42,6 | – | 1,2 | C | 1,0 | 440 | – | 80 | 28,4 | 42,6 | – | 1,2 | C | 1,0 | 440 | – | 70/3,1–143 |
| 30 | 28,4 | 42,6 | – | 0,6 | C | 1,0 | 440 | 10 | 80 | 28,4 | 42,6 | – | 0,6 | C | 1,0 | 440 | 10 | 70/2,9–150 |
| 31 | 9,9 | 4,3 | – | TAE<br>0,0125<br>TPTA<br>0,05 | B | 0,1 | 58 | 3 | 46 | 89,5 | 38,3 | – | TAE<br>0,675<br>TPTA<br>0,45 | B | 0,9(c) | 450 | 27 | 70/2,6–145 |
| 32 | 69,6 | 29,8 | – | 1,05 | B | 1,4 | 616 | 21 | 48 | 29,8 | 12,8 | – | 0,45 | B | 0,6 | 264 | 9 | 70/3,4–95 |

Beispiel 33:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 440 ml tert.Butanol und 10 ml entionisiertes Wasser vorgelegt. Unter Rühren werden darin 35,5 g Acrylamid und 35,5 g Acrylsäure gelöst, und dann wird dieser Lösung 1,0 g Tetraallyloxyethan zugesetzt.

Im Tropftrichter werden 440 ml tert.Butanol und 10 ml entionisiertes Wasser vorgelegt, unter Rühren darin 35,5 g Acrylamid und 35,5 g Acrylsäure gelöst und der Lösung 1,0 g Tetraallyloxyethan und 1 g Azodiisobutyronitril zugesetzt.

Das Reaktionsgefäß wird unter Rühren und Einleiten eines schwachen Stickstoffstromes auf 50 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 55–65 °C an und füllt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird das Polymer durch Abdestillation des tert.Butanols getrocknet. Es wird ein lockeres weißes Pulver erhalten. Die Lösungen des Polymerisats in entmineralisiertem Wasser, die man durch Einrühren des Polymerisats in der gewünschten Prozentigkeit und Einstellen des pH-Wertes mit einer geeigneten Base auf pH 7–9 erhält, zeichnen sich durch eine hervorragende Klarheit aus. Es sind keine mikrogelartigen Strukturen erkennbar, die Lösungen sind vollkommen homogen.

Kenndaten:

0,1%ige Lösung, pH 8–8,5, 1070 mPas, Haake VT 23

0,2%ige Lösung, pH 8–8,5, 4600 mPas, Haake VT 23

0,5%ige Lösung, pH 8–8,5, 24 000 mPas, Haake VT 23

Als Basen zur Einstellung des pH-Wertes eignen sich beispielsweise Ammoniak, Natriumhydroxid, Kaliumhydroxid, Natrium- und Kaliumkarbonat, Natrium- und Kaliumhydrogenkarbonat, Natrium- und Kaliumphosphat, Natrium- und Kaliumborat. Sie können als Reinsubstanzen fest ($NH_3$ gasförmig) oder als Lösungen in den gängigen Konzentrationen eingesetzt werden.

Beispiel 34:

Das Beispiel 33 wird wiederholt, die Polymerisation wird jedoch bei 40 °C mit Bis-(4-tert.butylcyclohexyl)-peroxidicarbonat gestartet, und das ausgefallene Polymerisat wird abgesaugt und getrocknet. Es ist ein ausgezeichnetes Verdickungsmittel, dessen Lösungen klar und ohne Mikrogelstruktur sind. Es eignet sich daher besonders für kosmetische Zubereitungen.

Kenndaten:

0,1%ige Lösung, pH 8–8,5, 900 mPas, Haake VT 23

0,2%ige Lösung, pH 8–8,5, 6120 mPas, Haake VT 23

0,5%ige Lösung, pH 8–8,5, 36 400 mPas, Haake VT 23

Beispiel 35:

Das Beispiel 33 wird wiederholt, jedoch wird bei 30 °C polymerisiert. Es werden 143 g (~100% der Theorie) eines ausgezeichneten Verdickungsmittels für kosmetische Zwecke erhalten.

Kenndaten:

0,1%ige Lösung, pH 8–8,5, 2750 mPas, Haake VT 23

0,2%ige Lösung, pH 8–8,5, 11 600 mPas, Haake VT 23

0,5%ige Lösung, pH 8–8,5, 32 900 mPas, Haake VT 23

Beispiel 36:

In einem Polymerisationsgefäß, ausgestattet mit Rührer, Rücflußkühler, Thermometer, Gaseinleitungsrohr, Tropftrichter mit eigenem Rührer und elektrisch beheiztem Wasserbad werden 440 ml tert.Butanol und 10 ml entionisiertes Wasser vorgelegt. Unter Rühren werden darin 32 g Acrylamid, 32 g Acrylsäure und 7,1 g Vinylphosphonsäure gelöst, und dann wird dieser Lösung 1,0 g Tetraallyloxyethan zugesetzt.

Im Tropftrichter werden 440 ml tert.Butanol und 10 ml entionisiertes Wasser vorgelegt, unter Rühren darin 32 g Acrylamid, 32 g Acrylsäure und 7,1 g Vinylphosphonsäure gelöst und der Lösung 1 g Tetraallyloxyethan und 1 g Azodiisobutyronitril zugesetzt.

Das Reaktionsgefäß wird unter Rühren und Einleiten eines schwachen Stickstoffstromes auf 58 °C erwärmt und bei dieser Temperatur 1 g Azodiisobutyronitril zugesetzt. Etwa 10 bis 30 Minuten nach der Initiatorzugabe setzt die Polymerisation mit einem deutlichen Temperaturanstieg ein. Sobald die Temperatur im Reatkionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunden auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird das Polymer durch Abdestillation des tert.Butanols getrocknet. Es wird ein lockeres weißes Pulver erhalten. Die Lösungen des Polymerisats in entmineralisiertem Wasser, die man durch Einrühren des Polymerisats in der gewünschten Prozentigkeit und Einstellen des pH-Wertes mit einer geeigneten Base auf pH 7–9 erhält, zeichnen

sich durch eine hervorragende Klarheit aus. Es sind keine mikrogelartigen Strukturen erkennbar, die Lösungen sind vollkommen homogen.

Kenndaten:

0,1%ige Lösung, pH 8–8,5, 2750 mPas, Haake VT 23

0,2%ige Lösung, pH 8–8,5, 12 800 mPas, Haake VT 23

0,5%ige Lösung, pH 8–8,5, 30 000 mPas, Haake VT 23

Beispiel 37:

In einem Polymerisationsgefäß, ausgestattet mit Rührer, Rückflußkühler, Thermometer, Gaseinleitungsrohr, Tropftrichter mit eigenem Rührer und elektrisch beheiztem Wasserbad werden 90 ml tert.Butanol und 2 ml entionisiertes Wasser vorgelegt und 9,9 g Acrylamid, 4,2 g Acrylsäure und 0,15 g Tetraallyloxyethan als Vernetzer gelöst.

Im Tropftrichter werden 450 ml tert.Butanol und 18 ml entionisiertes Wasser vorgelegt und 89,5 g Acrylamid, 38,4 g Acrylsäure und 1,35 g Tetraallyloxyethan gelöst. In einem zweiten Tropftrichter werden 0,5 g Bis-(4-tert.-butyl-cyclohexyl)-peroxidicarbonat in 40 ml Butanol gelöst.

Das Reaktionsgefäß wird unter Rühren und Einleiten eines schwachen Stickstoffstromes auf 46 °C erwärmt und bei dieser Temperatur der vorgelegten Reaktantenmischung 0,5 g Bis-(4-tert.-butyl-cyclohexyl)-peroxidicarbonat zugefügt. Etwa 10 bis 30 Minuten nach der Initiatorzugabe setzt die Polymerisation mit einem deutlichen Temperaturanstieg ein. Sobald die Temperatur im Reaktionsgefäß um ca. 3 °C angestiegen ist, wird die Monomerlösung aus dem Tropftrichter innerhalb von 2 Stunden gleichmäßig zugetropft. Die Polymerisationstemperatur steigt innerhalb von 1 Stunde auf 65–70 °C an und fällt gegen Ende der Reaktion wieder. Das Polymerisat fällt aus der Lösung aus, und es wird eine weiße, sehr gut rührbare Masse erhalten. Nach beendetem Monomerenzulauf wird der Ansatz noch 2 Stunden bei 80 °C Innentemperatur gerührt.

Nach Abkühlen des Reaktionsgemisches auf 20 °C wird es durch Einleiten von gasförmigem Ammoniak neutralisiert. Dabei steigt die Temperatur auf ca. 40 °C an. Das Polymerisationsgefäß wird mit einem Homogenisiergerät ausgerüstet. Die Polymerisatdispersion wird durch mehrfaches Umpumpen über das Homogenisiergerät gut homogenisiert.

Die weitere Aufarbeitung erfolgt gemäß den Angaben im obigen Beispiel 6.

Man erhält eine 37gew.%ige Polymerisatdispersion mit folgenden Kenndaten:

70/2,4–>160.

In analoger Weise lassen sich auch die in der folgenden Tabelle 2 angegebenen erfindungsgemäßen Copolymerisate herstellen.

In den Beispielen 38 bis 44 werden als Mineralöl 134 g eines paraffinischen Kohlenwasserstoffgemischs vom Siedebereich 200–400 °C eingesetzt.

In den Beispielen 37 bis 41 werden nach Ende der Polymerisation vor der Neutralisation 20 g Wasser dem Reaktionsgemisch zugesetzt.

Im Beispiel 41 wird der Zulauf in vier Stunden zugeführt.

Bei den in Tabelle 2 angegebenen Beispielen wird der im Zulauf enthaltende Initiator stets in 40 ml tert.Butanol gelöst und durch einen separaten Tropftrichter zudosiert.

Tabelle 2

| Vorlage Nr. | AM (g) | AS (g) | Reg. (g) | TAE (g) | Initi. | Initi. (g) | t.BuOH (ml) | $H_2O$ (ml) | Temp. (°C) | Zulauf AM (g) | AS (g) | Reg. (g) | TAE (g) | Initi. | Initi. (g) | t.BuOH (ml) | $H_2O$ (ml) | Kenndaten |
|---|---|---|---|---|---|---|---|---|---|---|---|---|---|---|---|---|---|---|
| 37 | 9,9 | 4,2 | – | 0,15 | B | 0,5 | 90 | – | 48 | 89,5 | 38,4 | – | 1,35 | B | 0,5 | 450 | – | 70/2,2–146 |
| 38 | 9,9 | 4,2 | – | 0,15 | B | 0,5 | 90 | – | 48 | 89,5 | 38,4 | – | 1,35 | B | 0,5 | 450 | – | 70/1,6–150 |
| 39 | 9,9 | 4,2 | – | 0,15 | B | 0,5 | 180 | – | 46 | 89,5 | 38,4 | – | 1,35 | B | 0,5 | 360 | – | 70/1,65–128 |
| 40 | 9,9 | 4,2 | – | 0,15 | B | 0,5 | 270 | – | 46 | 89,5 | 38,4 | – | 1,35 | B | 0,5 | 270 | – | 70/2,5–96 |
| 41 | 9,9 | 4,2 | – | 0,15 | B | 0,5 | 270 | – | 46 | 89,5 | 38,4 | – | 1,35 | B | 0,5 | 270 | – | 70/2,1–122 |
| 42 | 9,9 | 4,2 | – | 0,15 | B | 0,5 | 270 | 9,2 | 46 | 89,5 | 38,4 | – | 1,35 | B | 0,5 | 270 | 10,8 | 70/1,7–110 |
| 43 | 9,9 | 4,2 | – | 0,15 | A | 0,5 | 270 | 9,2 | 65 | 89,5 | 38,5 | – | 1,35 | A | 0,5 | 270 | 10,8 | 70/2,04–129 |
| 44 | 9,9 | 4,2 | – | 0,15 | B | 0,5 | 270 | 2,0 | 46 | 89,5 | 38,5 | – | 1,35 | B | 0,5 | 270 | 18 | 70/2,0–108 |

Tabelle 3

| Bsp. Nr. | AS (g) | AM (g) | Monomer III (g) | TAE (g) | Initiator (g) | t-Bu-OH (ml) | $H_2O$ (ml) | Temp. (°C) | AS (g) | AM (g) | Monomer III (g) | TAE (g) | Initiator (g) | t-Bu-OH (ml) | $H_2O$ (ml) | Kenndaten (ml) |
|---|---|---|---|---|---|---|---|---|---|---|---|---|---|---|---|---|
| 45 | 21,3 | 42,6 | IBMA 7,1 | 0,75 | A 1,0 | 440 | 15 | 50 | 21,3 | 42,6 | IBMA 7,1 | 0,75 | A 1,0 | 440 | 15 | 70/2.77–155 |
| 46 | 21,3 | 46,15 | IBMA 3,55 | 0,75 | A 1,0 | 440 | 15 | 50 | 21,3 | 46,15 | IBMA 3,55 | 0,75 | A 1,0 | 440 | 15 | 70/2.70–154 |
| 47 | 21,3 | 48,2 | IBMA 1,5 | 0,75 | A 1,0 | 440 | 15 | 50 | 21,3 | 48,2 | IBMA 1,5 | 0,75 | A 1,0 | 440 | 15 | 70/3.00–150 |
| 48 | 21,3 | 39,76 | IBMA 7,1/ MAS-C18 2,84 | 0,75 | A 1,0 | 440 | 15 | 50 | 21,3 | 39,76 | IBMA 7,1/ MAS-C18 2,84 | 0,75 | A 1,0 | 440 | 15 | 70/2.90–151 |
| 49 | 11,36 | 17,04 | 0 | 0,15 | 0,2 Lau-royl-peroxid | 393 | 10 | 80 | 102,24 | 153,36 | 0 | 1,35 | 0,2 Lauroyl-peroxid | 477 | 18 | 70/2.42–127 |
| 50 | 21,3 | 46,86 | MAS-C18-Ester 2,84 | 0,75 | A 1,0 | 440 | 15 | 50 | 21,3 | 46,86 | MAS-C18-Ester 2,84 | 0,75 | A 1,0 | 440 | 15 | 70/2.68–158 |
| 51 | 4,26 | 9,69 | MAS-C18-Ester 0,28 | 0,15 | B 0,1 | 250 | 1 | 45 | 38,34 | 87,21 | MAS-C18-Ester 2,52 | 1,35 | B 0,1 20mlBuOH | 290 | 4 | 70/2.11–145 |
| 52 | 4,26 | 8,52 | AS-Butyl-ester 1,42 | 0,13 | B 0,1 | 254 | 2 | 45 | 38,34 | 76,68 | AS-Butyl-ester 12,78 | 1,12 | B 0,1 20mlBuOH | 326 | 18 | 70/2.56–156 |
| 53 | 4,26 | 8,52 | AS-Butyl-ester 1,42 | 0,13 | B 0,1 | 254 | 0 | 45 | 38,34 | 76,68 | AS-Butyl-ester 12,78 | 1,12 | B 0,1 20mlBuOH | 326 | 0 | 70/2.30–115 |
| 54 | 5,68 | 5,02 | AS-Butyl-ester 3,5 | 0,15 | B 0,1 | 254 | 0 | 45 | 51,12 | 45,18 | AS-Butyl-ester 31,5 | 1,35 | B 0,1 20mlBuOH | 326 | 0 | 70/2.46–154 |
| 55 | 4,26 | 8,52 | AS-Butyl-ester 1,42 | 0,13 | B 0,1 | 254 | 0 | 45 | 38,34 | 76,68 | AS-Butyl-ester 12,78 | 1,12 | B 0,1 20mlBuOH | 326 | 0 | 70/2.08–156 |
| 56 | 11,36 | 17,04 | | 0,30 | B 0,2 | 508 | 0 | 45 | 102,24 | 153,36 | 0 | 2,70 | B 0,2 40mlBuOH | 612 | 28 | 70/2.09–128 |
| 57 | 11,36 | 17,04 | 0 | 0,40 | B 0,2 | 508 | 0 | 45 | 102,24 | 153,36 | 0 | 3,60 | B 0,2 40mlBuOH | 652 | 28 | 70/2.10–152 |

Tabelle 3 (Fortsetzung)

| Bsp. Nr. | AS (g) | AM (g) | Monomer III (g) | TAE (g) | Initiator (g) | t-Bu-OH (ml) | $H_2O$ (ml) | Temp. (°C) | AS (g) | AM (g) | Monomer III (g) | TAE (g) | Initiator (g) | t-Bu-OH (ml) | $H_2O$ (ml) | Kenndaten (ml) |
|---|---|---|---|---|---|---|---|---|---|---|---|---|---|---|---|---|
| 58 | 11,36 | 17,04 | 0 | 2,00 | B 0,2 | 508 | 10 | 45 | 102,24 | 153,36 | 0 | 0 | B 0,2 40mlBuOH | 652 | 18 | 70/1.97–141 |
| 59 | 22,72 | 34,08 | 0 | 0,60 | B 0,4 | 616 | 0 | 45 | 90,88 | 136,32 | 0 | 2,40 | 0 | 544 | 28 | 70/1.98–138 |
| 60 | 11,36 | 17,04 | 0 | 2,50 | B 0,2 | 508 | 0 | 45 | 102,24 | 153,36 | 0 | 0 | B 0,2 40mlBuOH | 652 | 28 | 70/2.05–156 |
| 61 | 11,36 | 17,04 | 0 | 1,50 | 0,2 Lau-royl-peroxid | 508 | 10 | 80 | 102,24 | 153,36 | 0 | 0 | 0,2 Lauroyl-peroxid | 652 | 18 | 70/2.40–117 |
| 62 | 11,36 | 17,4 | 0 | 0,30 | B 0,2 | 508 | 3 | 45 | 102,24 | 153,36 | 0 | 2,70 | B 0,2 40mlBuOH | 612 | 25 | 70/2.15–138 |

Durch Variation der Comonomeren, des Vernetzergehaltes, des Initiatorgehaltes und des Wassergehaltes lassen sich die Polymerisate der Tabelle 3 erhalten. Die Aufarbeitung erfolgt nach Beispiel 6. Folgende Abkürzungen wurden in Tabelle 3 zusätzlich verwandt: IBMA ≙ Isobutoxymethylenacrylamid, MAS-C18 ≙ Methacrylsäurestearylester, AS-Butylester ≙ Acrylsäurebutylester.

Die folgenden Beispiele illustrieren die Einsatzmöglichkeiten der vorstehenden Copolymerisate zur Herstellung von technischen, kosmetischen und pharmazeutischen Präparaten. Dies sind beispielsweise emulsionsartige Haut- und Haarpflegemittel, Zahnpasten, Rasiercremes, Sonnenschutzpräparate, Insektenschutzmittel oder Haarverformungspräparate. Weitere Einsatzgebiete sind bei der Rezeptierung bzw. Herstellung von pharmazeutischen Zubereitungen in Form von Tabletten, Salben und Gelen gegeben. Außerdem können die Copolymerisate aufgrund ihrer konsistenzerhöhenden Eigenschaften in vorzugsweise lösungsmittelhaltigen technischen Präparaten, wie Kraftfahrzeugreiniger, Motorenreiniger und Haushaltspräparaten verwendet werden.

Der mengenmäßige Anteil der vorzugsweise in neutralisierter Form eingesetzten Copolymerisate liegt, in Abhängigkeit von der gewünschten Viskosität, zwischen 0,02 und 3%, vorzugsweise zwischen 0,3 und 2%, bezogen auf das Gewicht der Fertigpräparate.

Die meist in nichtneutralisierter Form vorliegenden Polymerisate werden, in an sich bekannter Weise, mit dem Wasser bzw. wasserhaltigen Lösungsmittel gemischt, dispergiert und anschließend mit den gängigen Neutralisationsmitteln, wie Natronlauge, Kalilauge, Alkanolaminen, Ammoniumhydroxyd, Fettaminen oder deren Mischungen, auf einen pH-Wert zwischen 7 und 10 eingestellt, gegebenenfalls unter Erwärmen. Die Zugabe der weiteren Komponenten erfolgt vor oder nach der Neutralisation, sinnvollerweise vor der Neutralisation.

Die nachstehenden Beispiele sollen die Einsatzmöglichkeit der polymeren Verdicker veranschaulichen.

Ultraschall-Diagnose-Gel

0,7% Polymerisat gemäß Beispiel 34
0,15% NaOH
5,0% Glycerin
ad 100% Wasser + Konservierungsmittel

Salbe mit Zinkoxid

0,8% Polymerisat gemäß Beispiel 34
1,0% Triethanolamin
12,0% Zinkoxid
as 100% Wasser + Konservierungsmittel

Möbelpolitur

0,4% Polymerisat gemäß Beispiel 34
0,15% NaOH
5,0% Silikonölemulsion (30%ig)
3,0% Carnauba-Wachs Emulsion (20%ig)
as 100% Wasser

Haushaltreinigungsmittel

1,0% Polymerisat gemäß Beispiel 34
1,25% Triethanolamin
10,0% Isopropylalkohol
10,05% Nonylphenol + 10 mol EO
ad 100% Wasser

W/O-Creme
0,3% Polymerisat gemäß Beispiel 34
0,1% Monoethanolamin
4,0% Diglycerinsesquiisostearat
10,0% Paraffinöl
5,0% Cetylalkohol
2,0% Mikrowachs
0,2% Parfümöl
ad 100% Wasser + Konservierungsmittel

Nachrasurgel
1,0% Polymerisat gemäß Beispiel 34
0,32% Monoethanolamin
35,0% Ethylalkohol
0,1% Menthol
ad 100% Wasser + Konservierungsmittel

Haarwaschmittel
0,5% Polymerisat gemäß Beispiel 34
0,62% Triethanolamin
12,0% Cocosfettalkohol + 10 mol EO
0,1% Parfümöl
ad 100% Wasser + Konservierungsmittel

Haarfestigergel
1,2% Polymerisat gemäß Beispiel 34
1,5% Triethanolamin
40,0% Ethylalkohol
0,1% Parfümöl
ad 100% Wasser + Konservierungsmittel

Flüssige O/W-Emulsionen
0,20% Polymerisat gemäß Beispiel 34
0,05% NaOH
5,0% Isopropylpalmitat
5,0% Paraffinöl
5,0% Diglycerinstearat + 4 mol EO
0,1% Parfümöl
ad 100% Wasser + Konservierungsmittel

O/W-Creme
0,6% Polymerisat gemäß Beispiel 34
0,75% Triethanolamin
5,0% Vaseline
5,0% Sojaöl
3,0% Glycerinmonostearat
3,0% Tri-stearyltetraglykolether-ortho-Phosphorsäureester
ad 100% Wasser + Konservierungsmittel

Flüssige W/O-Emulsionen
0,2% Polymerisat gemäß Beispiel 34
0,6% Ammoniumhydroxyd (10%ig)
3,0% hydr. Rizinusöl + 7 mol EO
6,0% ®Hostacerin WO (Hoechst AG)
10,0% Isopropylpalmitat
15,0% Perhydrosqualen
ad 100% Konservierungsmittel + Wasser

Vergleichsbeispiel I

Das folgende Vergleichsbeispiel und seine in der Tabelle 3 angegebenen Variationen zeigen, daß bei der üblichen Durchführung der Polymerisation, bei der die Gesamtmenge aller Ausgangsmaterialien gemischt und dann die Reaktion eingeleitet wird, zwar auch durch Variation der Reaktionsbedingungen eine Variation der Polymerisateigenschaften möglich ist, daß es aber nicht gelingt, Produkte herzustellen, in denen eine hohe Wirksamkeit mit einer niedrigen Elektrolytempfindlichkeit in so vorteilhafter Weise kombiniert ist, wie in den erfindungsgemäß hergestellten Produkten.

In einem Reaktionsgefäß, ausgestattet mit Rührer, Rückflußkühler, Thermometer, Gaseinleitungsrohr, Tropftrichter und elektrisch beheiztem Wasserbad werden 440 ml tert.Butanol und 15 g Wasser, entionisiert, vorgelegt. Unter Rühren werden darin 49,7 g Acrylamid und 21,3 g Acrylsäure gelöst. Der Lösung wird 1 g Tetraallyloxyethan zugesetzt. Unter Einleiten eines schwachen Stickstoffstromes wird die Monomerenlösung gerührt und die Temperatur auf 50 °C gebracht. Bei dieser Temperatur wird 1 g Azodiisobutyronitril zugesetzt. Etwa 10–30 Minuten nach Katalysatorzugabe setzt die Polymerisation ein. Das Polymere fällt dabei als weiße breiartige Masse aus. Die Temperatur steigt im Verlauf von 30–40 Minuten auf 70–80 °C an. Nach beendeter Polymerisation, d. h., wenn die Reaktionstemperatur absinkt, wird noch 2 Stunden unter Rühren bei 80 °C Innentemperatur nachgeheizt.

Nach Abkühlen des Reaktionsgemisches auf 20 °C wird es durch Einleiten von gasförmigem Ammoniak neutralisiert. Dabei steigt die Temperatur auf ca. 40 °C an. Der Reaktionskolben wird mit einem Homogenisiergerät ausgerüstet. Die Polymerisatdispersion wird durch mehrfaches Umpumpen über das Homogenisiergerät fein homogenisiert.

Der Rückflußkühler wird durch eine Destillationsbrücke ersetzt, durch den Tropftrichter wird die Lösung von 3,5 g eines Sorbitanfettsäureesters, 4,9 g eines ethoxilierten Nonylphenols sowie 1,4 g eines Ethylenoxid-Propylenoxid-Blockpolymerisats in 119,5 g eines isoparaffinischen Kohlenwasserstoffgemisches vom Siedebereich 150 bis 350 °C zugetropft. Dabei wird das Reaktionsgemisch auf Siedetemperatur erhitzt und das Lösungsmittel tert.Butanol während des Zutropfens vollständig abdestilliert. Zurück bleiben ca. 207 g (~100% der Theorie) einer gießbaren, nicht absetzenden ca. 37%igen Dispersion des Polymers in dem Kohlenwasserstoffgemisch.

Die erhaltene Dispersion hat folgende Wirkungsdaten:

70/3,7–125.

Nach gleichem Verfahren werden die in Tabelle 4 aufgeführten Polymerisate hergestellt. Bei den Polymerisaten V–IX wird die Menge der Monomeren, des tert.Butanols, des Wassers, des zur Neutralisation eingesetzten Ammoniaks, der Emulgatoren sowie des Kohlenwasserstoffgemisches verdoppelt.

Tabelle 4

Vorlage (Kein Zulauf)

| Nr. | AM (g) | AS (g) | TAE (g) | t.BuOH (ml) | $H_2O$ (g) | Init. (g) | Temp. (°C) | Kenndaten |
|---|---|---|---|---|---|---|---|---|
| II | 49,7 | 21,3 | 1 | 440 | 15 | 0,5 | 50 | 70/3,4–155 |
| III | 49,7 | 21,3 | 1 | 440 | 15 | 3 | 50 | 70/4,4–120 |
| IV | 49,7 | 21,3 | 1 | 440 | 15 | 1 | 60 | 70/4,0–> 160 |
| V | 99,4 | 42,6 | 1 | 880 | 30 | 2 | 50 | 70/8,3–66 |
| VI | 99,4 | 42,6 | 1,25 | 880 | 30 | 2 | 50 | 70/5,5–75 |
| VII | 99,4 | 42,6 | 1,5 | 880 | 30 | 2 | 50 | 70/3,7–108 |
| VIII | 99,4 | 42,6 | 1,5 | 880 | 30 | 3 | 50 | 70/6,2–145 |
| IX | 99,4 | 42,6 | 1,5 | 880 | 30 | 1 | 50 | 70/5,5–135 |

Die obigen Vergleichsbeispiele zeigen, daß selbst die günstigsten herkömmlich hergestellten Produktvarianten I, II und VII bei weitem nicht die Qualität der erfindungsgemäß hergestellten Copolymerisate erreichen. Das herkömmlich hergestellte Copolymerisat IV hat nur noch die Qualitätsdaten 70/4–> 160 und ist insbesondere wegen seiner höheren Elektrolytempfindlichkeit unbrauchbar, die Copolymerisatdispersionen III, V, VI und VIII bis X ergeben selbst bei Einsatz von mehr als 4,2 Gew.% nicht mehr die Viskosität von 70 dPas in elektrolythaltiger Lösung und scheiden daher wegen zu geringer Wirksamkeit für den praktischen Einsatz aus.

In der Graphik Fig. 1 sind die oben angegebenen charakteristischen Wirkungsdaten der Produkte der Ausführungsbeispiele 1 bis 12 und der Vergleichsbeispiele I bis X dargestellt. Bei dieser Darstellung sind die Produkte umso günstiger, je weiter links und je weiter unten sich ihre Markierungen befinden. Man erkennt sofort die drastischen Qualitätsunterschiede zwischen den erfindungsgemäß und den herkömmlich hergestellten Produkten.

Die herkömmlichen Produkte sind in Doppelkreise gesetzt, die mit ihnen bezüglich ihrer chemischen Zusammensetzung direkt vergleichbaren erfindungsgemäßen Produkte in einfache Kreise.

Erfindungsgemäße Produkte mit abweichender chemischer Zusammensetzung stehen in Quadraten. Die mit einem Pfeil versehenen herkömmlichen Produkte fallen nicht mehr in den Darstellungsbereich des Diagramms.

## Patentansprüche

1. Verfahren zur Herstellung von Copolymerisaten, die ganz oder überwiegend aus Acrylamid und Acrylsäure bzw. Methacrylsäure aufgebaut sind, dadurch gekennzeichnet, daß man zur Herstellung von 100 Gew.-Teilen des Copolymerisats

10–90 Gew.-Teile Acrylamid (Monomer I)

90–10 Gew.-Teile Acrylsäure und/oder Methacrylsäure (Monomer II) oder eines Salzes dieser Säuren mit dem Kation $M^+$

0–40 Gew.-Teile eines copolymerisierbaren Monomeren der Formel III

$$R^3_a\text{–}CH_{2-a}=\underset{\displaystyle X}{\underset{|}{CH_{1-b}}}\text{–}R^3_b \qquad \text{(III)}$$

und

0–5 Gew.-Teile eines copolymerisierbaren, zwei oder mehr olefinische Doppelbindungen aufweisenden, bekannten Vernetzers

in tert.-Butanol bei Temperaturen zwischen 20 und 120 °C nach Art einer Fällungspolymerisation so polymerisiert, daß man

10–90 Gew.% Des Alkohols

10–90 Gew.% der Gesamtmenge der Monomeren I bis III und

10–100 Gew.% der Vernetzersubstanz

vorlegt, die Polymerisation mit

10–100 Gew.% des Initiators startet und den sich auf 100% ergänzenden Rest von Lösungsmittel, Monomeren, Vernetzer und Initiator nach Anspringen der Polymerisation einzeln oder im Gemisch miteinander im Verlauf von 1–10 h zudosiert wobei in der oben angegebenen Formel III der Comonomeren

$R^3$ Wasserstoff oder Methyl,

a und b jeweils die Werte 0 oder 1, bedeuten, wobei die Summe a + b ebenfalls 0 oder 1 ist, und

X eine Gruppe der Formel IV

|
$N–R^4$
|
$COR^5$ (IV)

worin $R^4$ und $R^5$ unabhängig voneinander für Wasserstoff, Alkyl mit 1 bis 4 Kohlenstoffatomen oder gemeinsam für Trimethylen oder Pentamethylen stehen und wobei für $R^4$ auch Methylol stehen kann, sofern $R^5$ Alkyl ist; Alkoxycarbonyl mit 1 bis 20 Kohlenstoffatomen; Hydroxyalkoxycarbonyl mit 1 bis 3 Kohlenstoffatomen; N-Methylolcarbonamid der Formel

$$HOCH_2NH–CO–,$$

dessen Methylolgruppe gegebenenfalls mit Alkanolen mit 1 bis 4 Kohlenstoffatomen verethert sein kann; Cyan; Phenyl oder Benzyl; mit 1 oder 2 Substituenten substituiertes Phenyl oder Benzyl;

Imidazolyl-(1); die Sulfonsäuregruppe; Sulfoalkylamidocarbonyl mit 1 bis 4 Kohlenstoffatomen im Alkylrest; die Phosphonsäuregruppe, wobei die Sulfonsäure- und Phosphonsäuregruppen auch in Form ihrer Salze mit einem Kation $M^+$ vorliegen können; die Phosphonsäureestergruppe der Formel V

O
‖
$-P-OR^6$ (V)
|
$O^{\ominus}M^{\oplus}$

worin $R^6$ Alkyl mit 1 bis 4 Kohlenstoffatomen ist; einen Rest der Formel VI

O
‖
$-COOCH_2CH_2-O-P-R^7$ (VI)
|
$R^8$

worin $R^7$ und $R^8$ gleich oder verschieden sind und für Alkyl mit 1 bis 7 Kohlenstoffatomen stehen; einen Rest der Formel VII

$-COO-C_pH_{2p}-N<^{R^7}_{R^8}$ (VII)

worin $R^7$ und $R^8$ die oben angegebenen Bedeutungen haben und p für eine Zahl von 1 bis 4 steht; oder einen Rest der Formel VIII

$-CONH-C_pH_{2p}-N<^{R^9}_{R^{10}}$ (VIII)

worin $R^9$ oder $R^{10}$ gleich oder verschieden sind und für Alkyl mit 1 bis 4 Kohlenstoffatomen stehen und p für eine Zahl von 1 bis 4 steht; sowie die den Formeln VII und VIII entsprechenden quaternisierten Gruppen bedeutet, wobei mindestens 50% der Grundkettenbausteine solche Reste X aufweisen, die hydrophilen Charakter besitzen und mindestens etwa 2% der Reste X saure Gruppen aufweisen bzw. deren Salze mit dem Kation $M^+$, wobei sich $M^+$ prinzipiell von jeder wasserlöslichen bekannten Base ableiten kann, deren Stärke ausreicht, die Sulfongruppen bzw. Carboxylgruppen der erfindungsgemäßen vernetzten Copolymerisate zu neutralisieren und die deren Hydrophilie nicht beeinträchtigt.

2. Verfahren gemäß Anspruch 1, dadurch gekennzeichnet, daß Comonomere der Formel III eingesetzt werden, in denen a = 0 und b = 1 ist, die somit der Formel IX

$R^3$
|
$CH_2=C-X$ (IX)

entsprechen und in denen $R^3$ Wasserstoff oder Methyl ist und

X eine Gruppe der Formel IV

|
$N-R^4$ (IV)
|
$COR^5$

worin $R^4$ und $R^5$ unabhängig voneinander für Wasserstoff, Methyl oder Ethyl oder gemeinsam für Trimethylen oder Pentamethylen stehen; Alkoxycarbonyl mit 1 bis 20 C-Atomen; Hydroxyalkoxycarbonyl mit 2 oder 3 C-Atomen; die Sulfonsäuregruppe; Sulfoalkylamidocarbonyl mit 1 bis 4 C-Atomen im Alkylrest,

die Phosphonsäuregruppe, wobei die Sulfonsäure- und Phosphonsäuregruppen auch in Form ihrer Alkali- oder Ammoniumsalze vorliegen können; und die Phosphonsäureestergruppe der Formel V

O
‖
$-P-OR^6$ (V)
|
$O^{\ominus}M^{\oplus}$

worin $R^6$ die im Anspruch 1 genannten Bedeutungen hat, bedeutet.

3. Verfahren gemäß den Ansprüchen 1 und 2, dadurch gekennzeichnet, daß man

30–70 Gewichtsteile Acrylamid,
70–30 Gewichtsteile Acrylsäure und/oder Methacrylsäure,
0–20 Gewichtsteile von copolymerisierbaren Monomeren

der Formel III bzw. IX copolymerisiert.

4. Verfahren gemäß den Ansprüchen 1 bis 3, dadurch gekennzeichnet, daß man

10 bis 50 Gew.% der Monomeren I bis III und
10 bis 100 Gew.% der Vernetzersubstanz, gelöst in
10 bis 75 Gew.% des Alkohols, vorlegt und nach dem Starten der Polymerisation mit 10 bis 100 Gew.% des Initiators den Rest von Alkohol, Monomeren, Vernetzer und Initiator zudosiert.

5. Verfahren gemäß Anspruch 4, dadurch gekennzeichnet, daß man 10 bis 20 Gew.% der Monomeren I bis III, gelöst in 10 bis 50 Gew.% des Alkohols, vorlegt und den Rest von Lösungsmittel und Monomeren zudosiert.

6. Verfahren gemäß Anspruch 1, dadurch gekennzeichnet, daß man die Copolymerisation in Gegenwart von bis zu 10 Gew.% Wasser, bezogen auf die Menge des Alkohols, vornimmt.

7. Verfahren gemäß den Ansprüchen 1 bis 6, dadurch gekennzeichnet, daß man einen gegebenenfalls einzupolymerisierenden Vernetzer zu 10 bis 50 Gew.% seiner Gesamtmenge vorlegt und den Rest zudosiert.

8. Verwendung der gemäß den Ansprüchen 1 bis 7 hergestellten Copolymerisate als Verdicker für technische, kosmetische und pharmazeutische Zubereitungen.

9. Verwendung der gemäß Anspruch 6 hergestellten Copolymerisate als Verdicker für kosmetische und pharmazeutische Zubereitungen.

## Claims

1. Process for preparing copolymers which are completely or predominantly built up from acrylamide and acrylic acid or methacrylic acid, characterized in that for preparing 100 parts by weight of the copolymer 10–90 parts by weight of acrylamide (monomer I), 90–10 parts by weight of acrylic acid and for methacrylic acid (monomer II) or of a salt of these acids with the cation $M^+$, 0–40 parts by weight of a copolymerizable monomer of the formula III

$$R^3_a-CH_{2-a}=\underset{\displaystyle X}{\underset{|}{CH_{1-b}}}-R^3_b \qquad (III)$$

and 0–5 parts by weight of a copolymerizable, known crosslinking agent which has two or more olefinic double bonds, are polymerized in tert. butanol at temperatures between 20 and 120 °C by precipitation polymerization such that 10–90% by weight of the alcohol, 10–90% by weight of the total amount of monomers I to III and 10–100% by weight of the crosslinker substance are initially mixed, polymerization is started with 10–100% by weight of the initiator and the remainder adding up to 100% of solvent, monomers, crosslinker and initiator are metered in in the course of 1 to 10 hrs after the polymerization has begun as individual components or as a mixture with one another, wherein in the formula III of the comonomers indicated above $R^3$ is hydrogen or methyl, a and b each are the value 0 or 1 with the sum of a and b each are the value 0 or 1 with the sum of a and b also being 0 or 1, and X is a group of the formula IV

$$\begin{array}{l} | \\ N-R^4 \\ | \\ COR^5 \end{array} \qquad (IV)$$

wherein $R^4$ and $R^5$ independently of one another denote hydrogen, alkyl having 1 to 4 C-atoms or together denote trimethylene or pentamethylene and wherein $R^4$ can also denote methylol if $R^5$ is alkyl; alkoxycarbonyl having 1 to 20 C-atoms; hydroxyalkoxycarbonyl having 1 to 3 C-atoms; N-methylolcarbonamide of the formula

$$HOCH_2NH-CO-,$$

the methylol group of which can optionally be etherified with alkanols having 1 to 4 C-atoms; cyano; phenyl or benzyl; phenyl or benzyl carrying 1 or 2 substituents; imidazol-1-yl; the sulphonic acid group; sulphoalkylamidocarbonyl having 1 to 4 C-atoms in the alkyl radical; the phosphonic acid group; it being also possible for the sulphonic acid and phosphonic acid groups to be present in the form of their salts with a cation $M^+$; the phosphonic acid ester group of formula V

$$\begin{array}{l} O \\ \| \\ -P-OR^6 \\ | \\ O^{\ominus}M^{\oplus} \end{array} \qquad (V)$$

wherein $R^6$ denotes alkyl having 1 to 4 C-atoms; a radical of the formula VI

$$-COOCH_2CH_2-O-\overset{\displaystyle O}{\overset{\|}{\underset{\displaystyle R^8}{\underset{|}{P}}}}-R^7 \qquad (VI)$$

wherein $R^7$ and $R^8$ are identical or different and denote alkyl having 1 to 7 C-atoms; a radical of the formula VII

$$-COO-C_pH_{2p}-N\langle^{R^7}_{R^8} \qquad (VII)$$

wherein $R^7$ and $R^8$ have the meanings indicated above and p is a number from 1 to 4; or a radical of the formula VIII

$$-CONH-C_pH_{2p}-N\langle^{R^9}_{R^{10}} \qquad (VIII)$$

wherein $R^9$ and $R^{10}$ are identical or different and denote alkyl having 1 to 4 C-atoms and p denotes a number from 1 to 4; as well as the quaternised groups corresponding to formulae VII and VIII, wherein at least 50% of the basic chain components have radicals X that are of hydrophilic character and at least about 25% of the radicals X have acid groups and/or their salts with the cation $M^+$, wherein $M^+$ can be principally derived from any watersoluble known base the strength of which suffices to neutralize the sulphonic groups or carboxylic groups of the crosslinked copolymers of the invention and which do not impair the hydrophilic character thereof.

2. The process of claim 1, characterized in that comonomers of the formula III are employed wherein a denotes 0 and b denotes 1, which thus correspond to the formula IX

$$CH_2=\overset{\displaystyle R^3}{\overset{|}{C}}-X \qquad (IX)$$

and in which $R^3$ is hydrogen or methyl and X denotes a group of the formula IV

$$\begin{array}{l} | \\ N-R^4 \\ | \\ COR^5 \end{array} \qquad (IV)$$

wherein $R^4$ and $R^5$ independently of one another denote hydrogen, methyl or ethyl, or together denote trimethylene or pentamethylene; alkoxycarbonyl having 1 to 20 C-atoms; hydroxyalkoxycarbonyl having 2 or 3 C-atoms; the sulphonic acid

group; sulphoalkylamidocarbonyl having 1 to 4 C-atoms in the alkyl radical; the phosphonic acid group, it being possible for the sulphonic acid and phosphonic acid groups to be also present in the form of their alkali metal or ammonium salts; and the phosphonic acid ester group of the formula V

O
‖
$-P-OR^6$ (V)
|
$O^{\ominus}M^{\oplus}$

wherein $R^6$ has the meanings indicated in claim 1.

3. The presence of claims 1 and 2, characterized in that 30–70 parts by weight of acrylamide, 70–30 parts by weight of acrylic acid and/or methylacrylic acid, 0–20 parts by weight of copolymerizable monomers of the formula III and/or IX are copolymerized.

4. The process of claims 1 to 3, characterized in that 10–50% by weight of the amount of monomers I to III and 10–100% by weight of the crosslinker substance, dissolved in 10–75% by weight of the alcohol, are initially mixed and after starting the polymerization with 10 to 100% by weight of the initiator the remainder of alcohol, monomer, crosslinker and initiator are metered in.

5. The process of claim 4, characterized in that 10–20% by weight of the monomers I to III, dissolved in 10 to 50% by weight of the alcohol, are initially mixed and the remainder of solvent and monomers are metered in.

6. The process of claim 1, characterized in that the copolymerization is carried out in the presence of up to 10% by weight of water relative to the amount of alcohol.

7. The process of claims 1 to 6, characterized in that 10 to 50% by weight of the total weight of a crosslinker which is optionally copolymerized is introduced as an initial charge and the remainder is metered in.

8. Use of the copolymers prepared according to claims 1 to 7 as thickeners for technical, cosmetic and pharmaceutical preparations.

9. Use of the copolymers prepared according to claim 6 as thickeners for cosmetic and pharmaceutical preparations.

**Revendications**

1. Procédé pour préparer des copolymères qui sont constitués, entièrement ou de façon prédominante, par de l'acrylamide et de l'acide acrylique ou de l'acide méthacrylique, procédé caractérisé en ce que, pour préparer 100 parties en poids de copolymères, on polymérise

10 à 90 parties en poids d'acrylamide (monomère I)
90 à 10 parties en poids d'acide acrylique et/ou d'acide méthacrylique (monomère II) ou d'un sel de ces acides avec le cation $M^+$
0 à 40 parties en poids d'un monomère copolymérisable, de formule III

$R^3_a-CH_{2-a}=CH_{1-b}-R^3_b$ (III)
|
X

et

0 à 5 parties en poids d'un agent connu de réticulation, copolymérisable, présentant deux ou plusieurs doubles liaisons oléfiniques,

dans du tertiobutanol à des températures comprises entre 20 et 120 °C, en opérant à la façon d'une polymérisation avec précipitation, en plaçant tout d'abord

10 à 90% en poids de l'alcool.
10 à 90% en poids de la quantité totale des monomères I à III et
10 à 100% en poids de l'agent de réticulation, en démarrant la polymérisation avec
10 à 100% en poids de l'amorceur et , après le démarrage de la polymérisation, en introduisant de façon dosée en une à dix heures le reste, pour compléter à 100%, du solvant, des monomères, de l'agent de réticulation et de l'amorceur, isolément ou en mélange, et, dans la formule III ci-dessus des comonomères,

$R^3$ représente un atome d'hydrogène ou un groupe méthyle, a et b valent chacun 0 ou 1, la somme (a + b) valant également 0 ou 1, et

X représente un groupe de formule IV:

|
$N-R^4$ (IV)
|
$COR^5$

où $R^4$ et $R^5$ représentent, indépendamment l'un de l'autre, un atome d'hydrogène, un groupe alkyle ayant 1 à 4 atomes de carbone, ou, ensemble, ils forment un groupe triméthylène ou pentaméthylène, et $R^4$ peut également représenter un groupe méthylol dans la mesure où $R^5$ est un groupe alkyle; $R^4$ et $R^5$ peuvent représenter chacun un groupe alcoxycarbonyle ayant 1 à 20 atomes de carbone, hydroxyalcoxycarbonyle ayant 1 à 3 atomes de carbone, N-méthylolcarboxamide de formule:

$HOCH_2NH-CO-$,

dont le groupe méthylol peut également être éthérifié avec des alcanols ayant 1 à 4 atomes de carbone; un groupe cyano, phényle ou benzyle, un groupe phényle ou benzyle portant un ou deux substituants; un groupe imidazolyl-(1); le groupe acide sulfonique; un groupe sulfoalkylamidocarbonyle ayant 1 à 4 atomes de carbone dans le reste alkyle; le groupe acide phosphonique, des groupes acides sulfoniques et acides phosphoniques pouvant également être présents sous forme de leurs sels avec un cation $M^+$; le groupe ester d'acide phosphonique, de formule V:

O
‖
$-P-OR^6$ (V)
|
$O^{\ominus}M^{\oplus}$

dans laquelle $R^6$ représente un groupe alkyle ayant 1 à 4 atomes de carbone; un reste de formule VI:

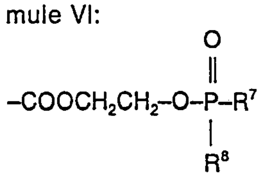

(VI)

dans laquelle $R^7$ et $R^8$ sont identiques ou différents et représentent chacun un groupe alkyle ayant 1 à 7 atomes de carbone; un reste de formule VII:

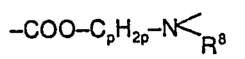

(VII)

dans laquelle $R^7$ et $R^8$ ont les sens indiqués ci-dessus, et p est un nombre valant 1 à 4; ou un reste de formule VIII:

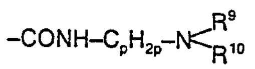

(VIII)

dans laquelle $R^9$ et $R^{10}$ sont identiques ou différents et représentent chacun un groupe alkyle ayant 1 à 4 atomes de carbone, et p est un nombre valant 1 à 4; ainsi que les groupes quaternisés correspondant à ceux présents dans les formules VII et VIII, au moins 50% des motifs de la chaîne de base présentant des restes X possédant un caractère hydrophile et au moins 2% environ des restes X présentant des groupes acides ou leurs sels avec le cation $M^+$, le cation $M^+$ pouvant en principe dériver de chaque base hydrosoluble connue, dont la force suffit pour neutraliser les groupes acides sulfoniques ou les groupes carboxyles des copolymères réticulés selon l'invention et qui ne nuit pas à leur caractère hydrophile.

2. Procédé selon la revendication 1, caractérisé en ce qu'on utilise des comonomères de formule III, dans laquelle a est nul et b vaut 1, et qui répondent donc à la formule IX:

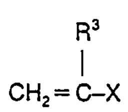

(IX)

et dans lesquels $R^3$ représente un atome d'hydrogène ou un groupe méthyle, et

X est un groupe de formule IV:

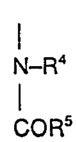

(IV)

dans laquelle $R^4$ et $R^5$ représentent, indépendamment l'un de l'autre, un atome d'hydrogène, un groupe méthyle ou éthyle ou forment ensemble un groupe triméthylène ou pentaméthylène; $R^4$ et $R^5$ peuvent également représenter un groupe hydroxyalcoxycarbonyle ayant 2 ou 3 atomes de carbone; le groupe acide sulfonique, un groupe sulfoalkylamidocarbonyle ayant 1 à 4 atomes de carbone dans le reste alkyle, le reste acide phosphonique, les groupes acides sulfoniques et acides phosphoniques pouvant également être présents sous forme de leurs sels alcalins ou d'ammonium; et le groupe ester d'acide phosphonique de formule V:

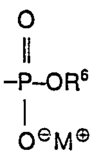

(V)

dans laquelle $R^6$ a le sens indiqué à la revendication 1.

3. Procédé selon les revendications 1 et 2, caractérisé en ce qu'on copolymérise

30 à 70 parties en poids d'acrylamide,

70 à 30 parties en poids d'acide acrylique et/ou d'acide méthacrylique,

0 à 20 parties en poids de monomères copolymérisables, répondant aux formules III et IX.

4. Procédé selon les revendications 1 à 3, caractérisé en ce qu'on place tout d'abord

10 à 50% en poids des monomères I à III, et

10 à 100% en poids de l'agent de réticulation, dissous dans

10 à 75% en poids de l'alcool, et, après le démarrage de la polymérisation que l'on obtient avec 10 à 100% en poids de l'amorceur, on introduit de façon dosée le reste de l'alcool, des monomères, de l'agent de réticulation et de l'amorceur.

5. Procédé selon la revendication 4, caractérisé en ce qu'on place tout d'abord 10 à 20% en poids des monomères I à III, dissous dans 10 à 50% en poids de l'alcool et l'on ajoute ensuite de façon dosée le reste du solvant et des monomères.

6. Procédé selon la revendication 1, caractérisé en ce qu'on effectue la copolymérisation en présence d'une quantité pouvant aller jusqu'à 10% en poids d'eau, sur la base de la quantité de l'alcool.

7. Procédé selon les revendications 1 à 6, caractérisé en ce qu'on place tout d'abord, à raison de 10 à 50% de sa quantité totale, un agent de réticulation à incorporer éventuellement par polymérisation, et l'on ajoute le reste de cet agent de façon dosée.

8. Utilisation des copolymères, préparés selon les revendications 1 à 7, comme épaississants pour des préparations techniques, cosmétiques et pharmaceutiques.

9. Utilisation des copolymères, produits selon l'exemple 6, comme épaississants pour des préparations cosmétiques et pharmaceutiques.

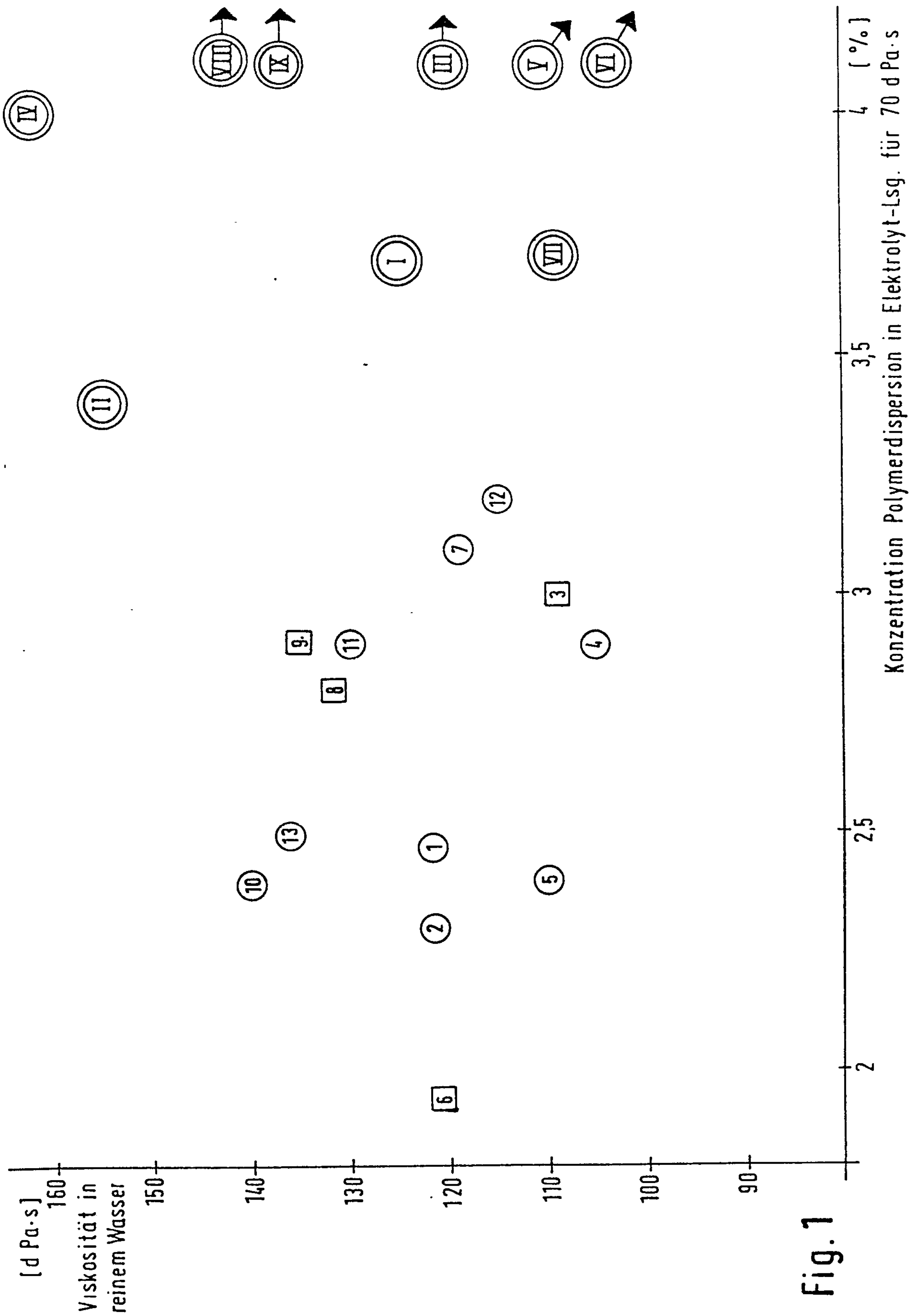
[d Pa·s]
Viskosität in reinem Wasser
160
150
140
130
120
110
100
90
2
2,5
3
3,5
4
[%]
Konzentration Polymerdispersion in Elektrolyt-Lsg. für 70 d Pa·s
1
2
3
4
5
6
7
8
9.
10
11
12
13
I
II
III
IV
V
VI
VII
VIII
IX

Fig. 1